# EUROPEAN PATENT APPLICATION

(11) **EP 2 617 435 A1**
(43) Date of publication of application: **24.07.2013**
(21) Application number: 11825214.7
(22) Date of filing: 14.09.2011
(51) Int. Cl.: A61K 45/00, A61K 31/517, A61K 31/5377, A61K 31/7105, A61K 48/00, A61P 29/00, A61P 37/06, A61P 37/08, C07D 239/94

(54) **THERAPEUTIC AGENTS AND PROPHYLACTIC AGENTS FOR SYMPTOMS ACCOMPANYING AUTOIMMUNE DISEASES, INFLAMMATORY DISEASES, ALLERGY DISEASES AND ORGAN TRANSPLANTS**

(30) Priority: 28.12.2010 JP 2010292031; 16.09.2010 JP 2010208534
(71) Applicant: Osaka University, Suita-shi, Osaka 565-0871 (JP)
(72) Inventor: HIRANO, Toshio, Suita-shi, Osaka 565-0871 (JP); MURAKAMI, Masaaki, Suita-shi, Osaka 565-0871 (JP)
(74) Representative: Vossius & Partner
(86) International application number: PCT/JP2011/071022
(87) International publication number: WO 2012/036214

(57) **Abstract**

A major object of the present invention is to provide a novel therapeutic or prophylactic agent for at least one disease or symptom selected from the group consisting of autoimmune diseases, inflammatory diseases, allergic diseases, and symptoms accompanying organ transplants; the therapeutic or prophylactic agent artificially controls the activity of IL-6 amplifier, thus controlling immune reactions in living organisms. Another object of the present invention is to provide an immunosuppressant, an inflammatory cytokine production inhibitor, and an IL-6 amplifier inhibitor.

The compound that inhibits expression of the function of a protein belonging to ErbB1 pathway provided as means for achieving the object is capable of suppressing the activity of IL-6 amplifier, thereby reducing production of inflammatory cytokine such as IL-6. Therefore, the compound that inhibits the function of a protein belonging to ErbB1 pathway can be used as an active ingredient of the therapeutic agent or the prophylactic agent for at least one disease or symptom selected from the group consisting of autoimmune diseases, inflammatory diseases, allergic diseases, and symptoms accompanying organ transplants.

SEQUENCE LISTING

<110>  OSAKA UNIVERSITY

<120>  MEDICAMENT FOR TREATMENT AND PROPHYLAXIS OF AUTOIMMUNE DISEASES,
       INFLAMMATORY DISEASES, AND SYMPTOMS ASSOCIATED WITH ORGAN
       TRANSPLANTS

<130>  P11-078

<150>  JP 2010-208534
<151>  2010-09-16

<150>  JP 2010-292031
<151>  2010-12-28

<160>  13

<170>  PatentIn version 3.4

<210>  1
<211>  20
<212>  DNA
<213>  Mus musculus

<400>  1
gtggcaggta gagcaggaag                                                  20


<210>  2
<211>  20
<212>  DNA
<213>  Mus musculus

<400>  2
ccacctgaaa ggcactctgt                                                  20


<210>  3
<211>  23
<212>  DNA
<213>  Mus musculus

<400>  3
gattagcgat gatgaaccag gtt                                              23


<210>  4
<211>  21
<212>  DNA
<213>  Mus musculus

<400>  4
cctcccatct ccttcatgac a                                                21


<210>  5
<211>  20
<212>  DNA
<213>  Mus musculus

<400>  5
acagtgtggg aagcaagtcc                                                  20

<210>  6
<211>  20
<212>  DNA
<213>  Mus musculus

<400>  6
ccgtgaactc ctttgaccat                                                    20


<210>  7
<211>  20
<212>  DNA
<213>  Mus musculus

<400>  7
ctgcctcttg ggtcttgacg                                                    20


<210>  8
<211>  23
<212>  DNA
<213>  Mus musculus

<400>  8
gcggtacagt tatcctcgga ttc                                                23


<210>  9
<211>  21
<212>  PRT
<213>  Mus musculus

<400>  9

Met Glu Val Gly Trp Tyr Arg Ser Pro Phe Ser Arg Val Val His Leu
1               5                   10                  15


Tyr Arg Asn Gly Lys
            20


<210>  10
<211>  20
<212>  DNA
<213>  Homo sapiens

<400>  10
ggagacttgc ctggtgaaaa                                                    20


<210>  11
<211>  20
<212>  DNA
<213>  Homo sapiens

<400>  11
gtcaggggtg gttattgcat                                                    20


<210>  12
<211>  21
<212>  DNA
<213>  Homo sapiens

<400>  12
ctgcctgggt ttccatcttc t                                                21

<210>  13
<211>  23
<212>  DNA
<213>  Homo sapiens

<400>  13
gccattcatg tcagagctac act                                              23

## Description

### Technical Field

The present invention mainly relates to a therapeutic agent and a prophylactic agent for diseases including autoimmune diseases, inflammatory diseases, and allergic diseases; and a therapeutic agent and a prophylactic agent for symptoms accompanying organ transplants. Further, the present invention relates to a method for screening an active ingredient of a therapeutic agent and a prophylactic agent for the diseases or symptoms.

### Background Art

Chronic inflammatory diseases due to uncontrollable inflammatory reactions occur in many patients, and have become a serious social problem. For example, it is known that at least 1% of the Japanese population becomes ill with an autoimmune disease, which is a chronic inflammatory disease. Although inflammatory reactions are often seen in living organisms, the mechanism of such uncontrollable inflammatory reactions induced by causative diseases is still not known. Inflammatory reactions are assumed to be triggered by a complicated combination of natural immune reactions locally caused by exogenous antigens, antigen-specific acquired immune responses, individual genetic backgrounds, environmental factors, and the like. Because these reactions are caused by the control of a significantly large number of molecules, the study of the mechanism at a molecular level or application of the mechanism to establish a therapeutic or prophylactic method has not yet been fully established.

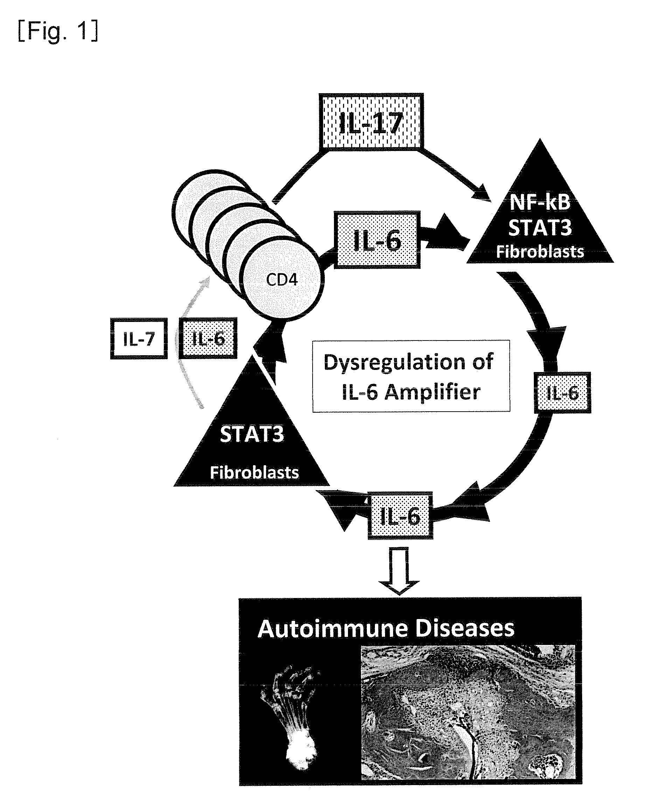

Cytokines designate a group of low-molecular-weight proteins, which are known to be produced in various cells in response to immune response so as to control the level of immune reaction. Inflammatory reactions are assumed to be caused by a cytokine, in particular, excessive production of a group of cytokines called inflammatory cytokine (such as interleukin-6 (IL-6)). Recent studies have discovered "Th-17 cells" that produce, upon activation, a large amount of IL-17, which is a kind of inflammatory cytokine. A report has revealed that Th-17 cells play an important role in many autoimmune diseases (rheumatoid arthritis, diabetes, autoimmune encephalomyelitis, etc.) (Non-patent Document 1). Further, another report has shown that the interaction between immune cells and non-immune cells such as fibroblasts is critically involved in the onset of autoimmune disease (Non-patent Document 2). Based on these findings, further studies were conducted, revealing the existence of "IL-17A-induced IL-6 production amplifier loop," in which IL-17A produced from Th-17 cells act on fibroblasts (type I collagen-positive cells) together with IL-6, thereby producing more IL-6, as well as chemokine that defines migration of immunocompetent cells (see Fig. 1). Studies have also shown that this dysregulation of IL-6 amplifier is critically involved in the onset and the disease progression of autoimmune arthritis (F759 arthritis) and autoimmune encephalomyelitis (EAE) in mice (Non-patent Document 1).

Based on the above findings, the possibility that artificial control of IL-6 amplifier activity may enable control of immune reactions in living organisms was suggested. In particular, artificial reduction in IL-6 amplifier activity is expected to enable treatment or prevention of autoimmune diseases. However, no specific method for controlling IL-6 amplifier activity has thus far been reported.

### Citation List

### Non-patent Documents

Non-patent Document 1
   Ogura H, Murakami M, Okuyama Y, Tsuruoka M, Kitabayashi C, Kanamoto M, Nishihara M, Iwakura Y, Hirano T. Immunity. 2008 Oct 17; 29(4):628-36.
Non-patent Document 2
   Sawa S, Kamimura D, Jin GH, Morikawa H, Kamon H, Nishihara M, Ishihara K, Murakami M, Hirano T. J Exp Med. 2006 Jun 12; 203(6):1459-70.
Non-patent Document 3
   Ohtani T, Ishihara K, Atsumi T, Nishida K, Kaneko Y, Miyata T, Itoh S, Narimatsu M, Maeda H, Fukada T, Itoh M, Okano H, Hibi M, Hirano T. Immunity. 2000 Jan; 12(1):95-105.
Non-patent Document 4
   Igarashi H, Hashimoto J, Tomita T, Yoshikawa H, Ishihara K. Clin Exp Immunol. 2010 Jul 1; 161(1):71-80.
Non-patent Document 5
   Sano S, Chan KS, DiGiovanni J. J Dermatol Sci. 2008 Apr; 50(1):1-14.

### Summary of Invention

### Technical Problem

A major object of the present invention is to provide a novel therapeutic or prophylactic agent that artificially controls the activity of IL-6 amplifier, thus controlling immune reactions in living organisms, thereby treating or preventing diseases and symptoms such as autoimmune diseases, inflammatory diseases, allergic diseases, or symptoms accompanying organ transplants; and a method for screening an active ingredient of the therapeutic or prophylactic agent. Another object of the present invention is to provide an immunosuppressant, an inflammatory cytokine production inhibitor and an IL-6 amplifier inhibitor.

### Solution to Problem

The inventors of the present invention conducted extensive research to solve the above objectives, and found that inhibiting expression of the function of a protein belonging to ErbB1 pathway results in a decrease in IL-6 production, which is assumed to be derived from significant suppression in the activity of IL-6 amplifier. The invention has been completed by making further improvement based on these findings.

Specifically, the present invention encompasses the following inventions:
[Item 1]
   A therapeutic agent or a prophylactic agent for at least one disease or symptom selected from the group consisting of autoimmune diseases, inflammatory diseases, allergic diseases, and symptoms accompanying organ transplants, the therapeutic agent or the prophylactic agent comprising a compound that inhibits expression of the function of a protein belonging to ErbB1 pathway.
[Item 2]
   The therapeutic agent or the prophylactic agent according to Item 1, wherein the protein belonging to ErbB1 pathway is at least one member selected from the group consisting of ErbB1 protein and a ligand protein of ErbB1 protein.
[Item 3]
   The therapeutic agent or the prophylactic agent according to Item 1 or 2, wherein the protein belonging to ErbB1 pathway is epiregulin.
[Item 4]
   The therapeutic agent or the prophylactic agent according to any one of Items 1 to 3, wherein the compound that inhibits expression of the function of the protein belonging to ErbB1 pathway is an antibody that specifically binds to the protein.
[Item 4-1]
   The therapeutic agent or the prophylactic agent according to any one of Items 1 to 3, wherein the compound that inhibits expression of the function of the protein belonging to ErbB1 pathway is an antibody that specifically binds to the protein, thereby inhibiting the function of the protein.
[Item 5]
   The therapeutic agent or the prophylactic agent according to any one of Items 1 to 3, wherein the compound that inhibits the function of the protein belonging to ErbB1 pathway is at least one RNA molecule selected from the group consisting of siRNA, shRNA, and miRNA that targets a gene encoding the protein or a vector capable of expressing the RNA molecule.
[Item 6]
   The therapeutic agent or the prophylactic agent according to Item 1 or 2, wherein the compound that inhibits the function of the protein belonging to ErbB1 pathway is a kinase inhibitor specific to ErbB1 protein.
[Item 7]
   The therapeutic agent or the prophylactic agent according to Item 6, wherein the compound that inhibits the function of the protein belonging to ErbB1 pathway is at least one member selected from the group consisting of PD153035, PD168393, gefitinib, and erlotinib.
[Item 8]
   The therapeutic agent or the prophylactic agent according to any one of Items 1 to 7, wherein the disease or symptom is caused by overexpression of inflammatory cytokine.
[Item 9]
   The therapeutic agent or the prophylactic agent according to any one of Items 1 to 8, wherein the disease or symptom is an autoimmune disease.
[Item 10]
   The therapeutic agent or the prophylactic agent according to any one of Items 1 to 9, wherein the autoimmune disease is at least one member selected from the group consisting of rheumatoid arthritis and multiple sclerosis.
[Item 11]
   An immunosuppressant comprising a compound that inhibits expression of the function of a protein belonging to ErbB1 pathway.
[Item 12]
   The immunosuppressant according to Item 11, wherein the immunosuppressant treats or prevents at least one disease or symptom selected from the group consisting of autoimmune diseases, inflammatory diseases, allergic diseases and symptoms accompanying organ transplants.
[Item 13]
   An inflammatory cytokine production inhibitor comprising a compound that inhibits expression of the function of a protein belonging to ErbB1 pathway.
[Item 14]
   An IL-6 amplifier inhibitor comprising a compound that inhibits expression of the function of a protein belonging to ErbB1 pathway.
[Item 15]
   A method for screening, among test substances, an active ingredient of a therapeutic agent or a prophylactic agent for at least one disease or symptom selected from the group consisting of autoimmune diseases, inflammatory diseases, allergic diseases, and symptoms accompanying organ transplants, the method comprising the steps of:
   (i) determining whether a test substance is a compound that inhibits expression of the function of a protein belonging to ErbB1 pathway; and
   (ii) selecting the test substance determined as the compound that inhibits expression of the function of the protein belonging to ErbB1 pathway in Step (i) as the active ingredient of the therapeutic agent or the prophylactic agent.
[Item 16]
   The method according to Item 15, wherein the protein belonging to ErbB1 pathway is at least one member selected from the group consisting of ErbB1 protein and a ligand protein of ErbB1 protein.
[Item 17]
   The method according to Item 15 or 16, wherein the protein belonging to ErbB1 pathway is epiregulin.
[Item 18]
   The method according to any one of Items 15 to 17, wherein the disease or symptom is an autoimmune disease.
[Item 19]
   The method according to any one of Items 15 to 18, wherein the autoimmune disease is at least one member selected from the group consisting of rheumatoid arthritis and multiple sclerosis.
[Item 20]
   The method according to any one of Items 15 to 19, wherein the test substances are either an antibody or a nucleic acid capable of suppressing expression of the protein.
[Item 21]
   A therapeutic method or a prophylactic method for at least one disease or symptom selected from the group consisting of autoimmune diseases, inflammatory diseases, allergic diseases, and symptoms accompanying organ transplants, the therapeutic method or the prophylactic method comprising a step administering a compound that inhibits expression of the function of a protein belonging to ErbB1 pathway.
[Item 22]
   A compound that inhibits expression of the function of a protein belonging to ErbB1 pathway, the compound being used for the treatment or prevention of at least one disease or symptom selected from the group consisting of autoimmune diseases, inflammatory diseases, allergic diseases, and symptoms accompanying organ transplants.
[Item 23]
   Use of a compound that inhibits expression of the function of a protein belonging to ErbB1 pathway for the manufacture of a therapeutic agent or a prophylactic agent for at least one disease or symptom selected from the group consisting of autoimmune diseases, inflammatory diseases, allergic diseases, and symptoms accompanying organ transplants.

### Advantageous Effects of Invention

The novel therapeutic or the prophylactic agent of the present invention that treats or prevents diseases and symptoms such as autoimmune diseases, inflammatory diseases, allergic diseases, or symptoms accompanying organ transplants; or the therapeutic or prophylactic agent for the above diseases or symptoms screened by the present invention is expected to artificially control the activity of IL-6 amplifier and control immune reaction of living organisms. Thereby, the present invention is expected to contribute to improvement in the technique for treating the above diseases or like diseases. Further, the novel immunosuppressant, inflammatory cytokine production inhibitor and IL-6 amplifier inhibitor of the present invention are conducive to improving the technique for treating the above diseases or like diseases, and can also serve as useful tools for the basic study of immunology. Therefore, these agents of the present invention are expected to improve the technique for treating the above diseases or like diseases, or to become a platform for development in the entire medical field.

### Brief Description of Drawings

[Fig. 1] A schematic view showing IL-6 amplifier.
[Fig. 2] (A) A schematic view showing the ErbB1 pathway. (B) A simplified view of HER family receptors.
[Fig. 3] A graph showing measurement results regarding ErbB1 expression suppression effect by shRNA in IL-6 amplifier. h7-1, h7-2 and h7-3 denote clones created by introducing different lentiviruses; the clones encode shRNA specific to ErbB1 gene.
[Fig. 4] Graphs showing measurement results regarding an effect of the presence of fetal bovine serum (FBS) in IL-6 amplifier. (A) A graph showing mIL-6 protein production amount and relative cell survival. (B) A graph showing IL6 mRNA expression as relative ratios with respect to HPRT gene expression amounts (control).
[Fig. 5] Graphs showing measurement results regarding effects of specific growth factors in IL-6 amplifier. The individual effects of (A) EGF protein, (B) HB-EGF protein, and (C) epiregulin protein are shown as production amounts of mIL-6 protein.
[Fig. 6] Graphs showing measurement results regarding effects of specific growth factors in IL-6 amplifier. The individual effects of (A) VEGF protein, (B) PDGF-CC protein, and (C) FGF7 protein are shown as production amounts of mIL-6 protein.
[Fig. 7] Graphs showing measurement results regarding an effect of addition of epiregulin protein on the expression amount of a target gene of IL-6 amplifier. Figs. 7(A) and 7(C) show the results for BC-1 cells, Figs. 7(B) and 7(D) show the results for MEF cells. Figs. 7(A) and (7B) show IL6 mRNA expression and Figs. 7(C) and 7(D) show Ccl20 mRNA expression as relative ratios with respect to a control group.
[Fig. 8] Graphs showing examination results for receptors of epiregulin protein upon activation of IL-6 amplifier. Effects of PD153035 (A) and ErbB2 Inhibitor II (B) are shown as production amounts of mIL-6 protein.
[Fig. 9] Graphs showing measurement results regarding an effect of activation of IL-6 amplifier on the expression amount of epiregulin gene. Figs. 9(A) and 9(C) show results for BC-1 cells,
Figs. 9(B) and 9(D) show results for MEF cells. The figures show expression amounts of mice epiregulin gene as relative ratios with respect to a control group.
[Fig. 10] A graph showing measurement results regarding relevance of epiregulin protein to IL-6 amplifier in the synovial cells derived from a human rheumatoid arthritis patient. The figures show expression amounts of hIL-6 gene as relative ratios with respect to a control group.
[Fig. 11] (A) An observation image of appearance of a F759/F759-STAT3^{fl}/^{fl}-K5 Cre mouse. A hematoxylin-eosin stained image of a skin tissue of (B) a F759/F759-STAT3^{fl/fl}-K5 Cre mouse and (C) a F759/F759-STAT3^{fl/fl} mouse.
[Fig. 12] Graphs showing measurement results regarding an effect of an ErbB1 inhibitor in IL-6 amplifier. An effect of PD153035 (A) in BC-1 cells, an effect of PD153035 (B) in MEF cells, an effect of PD168393 (C) in BC-1 cells, and an effect of PD168393 (D) in MEF cells are shown as production amounts of mIL-6 protein. As a control, an effect of a FGF RTK inhibitor (E) and an effect of a FGF/PDGF/VEGF RTK inhibitor (F) in BC-1 cells are shown as production amounts of mIL-6 protein.
[Fig. 13] Graphs showing measurement results regarding an effect of a PI3 kinase inhibitor in IL-6 amplifier. Effects of LY294002 (A) and PIK75 (B) are shown as production amounts of mIL-6 protein.
[Fig. 14] Images showing a method for evaluating arthritis symptom in the mouse malleolus. (A) A healthy state: clinical score = 0. (B) A state of severe arthritis: the movable range of malleolus joint is 60° smaller than the healthy case (clinical score = 3).
[Fig. 15] Graphs showing an effect of administration of an ErbB1 inhibitor in cytokine-induced arthritis mice. The individual effects of PD153035 (A) and PD168393 (B) are shown as clinical scores.
[Fig. 16] Graphs of clinical scores showing effects of shRNA (Ereg sh #1 to #3) and anti-epiregulin antibody (C) in epiregulin gene (A) in cytokine-induced arthritis mice. Fig. 16(B) shows a knockdown efficiency of shRNA with respect to epiregulin gene as relative epiregulin mRNA expression. The horizontal axis in Figs. 16(A) and 16(C) denotes the time (days) passed after the injection.
[Fig. 17] A simplified view showing a method for producing an experimental autoimmune encephalomyelitis (EAE) mouse.
[Fig. 18] Graphs of clinical scores showing effects of an antibody (anti-epiregulin) (A) that specifically binds to epiregulin and gefitinib (product name: Iressa) (B) with respect to EAE mice. The horizontal axis denotes the time (days) passed after pathogenic Th17 cell injection.
[Fig. 19] Graphs showing clinical scores of effects of shRNA (Egfr sh #1 to #3) in ErbB1 (Egfr) gene in cytokine-induced arthritis mice. The horizontal axis denotes the time (days) after the injection.
[Fig. 20] Graphs of clinical scores showing an effect of gefitinib (product name: Iressa) in cytokine-induced arthritis mice. The horizontal axis denotes the time (days) after the injection.
[Fig. 21] Graphs showing epiregulin gene expression suppression effect given by shRNA in IL-6 amplifier, as production amounts of amIL-6 protein. In the figures, the white bar denotes a case in which epiregulin protein was not added (Epiregulin (-)), and the black bar denotes a case in which epiregulin protein was added (Epiregulin (+)). "mock" shows results for a clone that expresses non-specific shRNA, and "sh-Ereg" shows results for a clone that expresses shRNA specific to epiregulin gene. Fig. 21(A) shows a production amount of mIL6 protein 48 hours after the culture, and
Fig. 21(B) shows a production amount of mIL6 protein 72 hours after the culture.
[Fig. 22] Fig. 22(A) shows measurement results regarding effect of addition of epiregulin on hIL6 mRNA expression in a human synovial fibroblast. The measurement results are shown as relative ratios with respect to a control group. Fig. 22(B) shows measurement results regarding the effect of ErbB1 inhibitor PD153035 in IL-6 amplifier in a human synovial fibroblast. The measurement results are shown as relative ratios of hIL6 mRNA expression with respect to a control group.
[Fig. 23] A graph showing measurement results regarding an effect of activation of IL-6 amplifier on the expression amount of epiregulin gene in a human synovial fibroblast. The expression amounts of human epiregulin genes are shown as relative ratios with respect to a control group.

In the above figures, "NS" denotes "no significance," "*" denotes "significance" where p value<0.05 according to the t-test, "**" denotes "significance" where p value<0.01 according to the t-test, and "***" denotes "significance" where p value<0.001 according to the t-test.

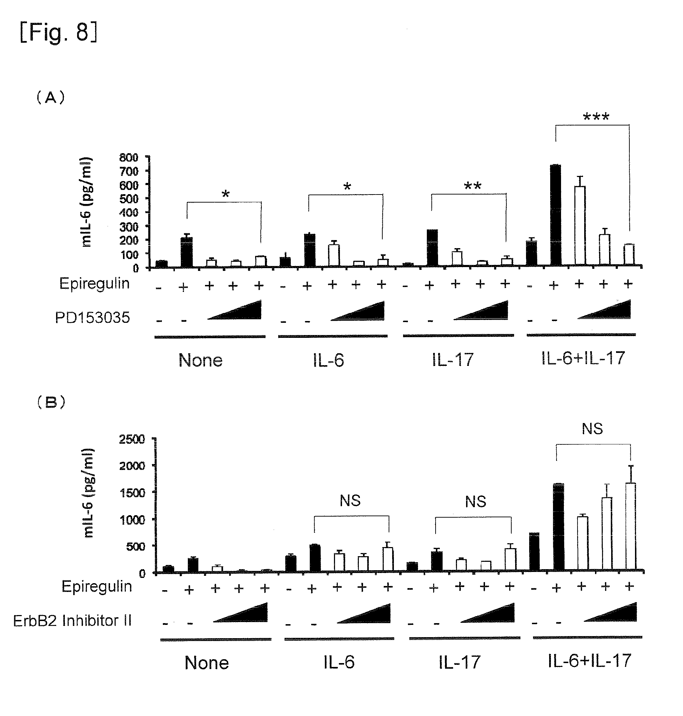

In the above figures, "-" denotes no addition, and "+" denotes addition. In Figs. 8, 12, and 13, the addition amounts of the inhibitors increase from left to right.

### Description of Embodiments

### (1) Therapeutic Agent or Prophylactic Agent

The present invention relates to a therapeutic agent or prophylactic agent that treats or prevents diseases and symptoms such as autoimmune diseases, inflammatory diseases, and allergic diseases; or symptoms accompanying organ transplants. The therapeutic or prophylactic agent of the present invention contains a compound that inhibits expression of the function of a protein belonging to ErbB1 pathway. The therapeutic agent or the prophylactic agent of the present invention may be a therapeutic or prophylactic pharmaceutical composition for the above diseases or symptoms.

The diseases and symptoms treatable or preventable by the therapeutic or prophylactic agent of the present invention include diseases such as autoimmune diseases, inflammatory diseases, or allergic diseases, and symptoms accompanying organ transplants (these diseases and symptoms may hereinafter also be referred to as "the diseases, etc."). The therapeutic or prophylactic agent of the present invention can treat at least one of the above diseases and symptoms. Preferably, the diseases, etc., are autoimmune diseases. The diseases, etc., are accompanied by inflammation presumably induced by excessive production of cytokine (in particular, IL-1, IL-6, IL-17, TNF-α, more preferably, inflammatory cytokine such as IL-6) due to immune reaction with respect to antigen. Accordingly, the therapeutic or prophylactic agent of the present invention may treat or prevent inflammation accompanying autoimmune diseases, inflammatory diseases, allergic diseases and like diseases, or inflammation induced by organ transplants or the like. The diseases, etc., may be autoimmune diseases, inflammatory diseases, allergic diseases and like diseases, or symptoms accompanying organ transplants, which are caused by overexpression of cytokine (preferably inflammatory cytokine).

Among the above diseases, the autoimmune diseases include various morbid conditions accompanied by symptoms in various body parts. For example, the autoimmune diseases may include, but are not limited to, autoimmune diseases of specific organs including autoimmune diseases of cranial nervous system, autoimmune diseases of blood circulatory system, and autoimmune diseases of bowel/digestive system. Examples of cranial nervous system autoimmune diseases include, but are not limited to, chronic inflammatory demyelinating polyneuropathy, and multiple sclerosis. Examples of blood circulatory system autoimmune diseases include, but are not limited to, arteriosclerosis. Examples of bowel/digestive system autoimmune diseases include, but are not limited to, Crohn's disease and ulcerative colitis. Obviously, autoimmune diseases of other body parts or organs, for example, chronic nephritis, chronic inflammatory pulmonary disease, etc. and systemic autoimmune diseases, for example, diabetes, rheumatoid arthritis, etc., are also included. The autoimmune diseases are preferably rheumatoid arthritis and multiple sclerosis, more preferably multiple sclerosis. The autoimmune diseases may be autoimmune diseases other than rheumatoid arthritis.

Among the above diseases, the inflammatory diseases can include various inflammatory conditions accompanied by symptoms in various body parts. For example, the inflammatory diseases may include any of alterative inflammation, exudative inflammation, and hyperplastic inflammation. The inflammatory diseases may also be acute or chronic; however, chronic inflammation is preferable. For example, the inflammatory diseases include, but are not limited to, inflammatory diseases of specific organs such as cranial nervous system inflammatory diseases, blood circulatory system inflammatory diseases, bowel/digestive system inflammatory diseases, and the like.

Among the above diseases, the allergic diseases are not particularly limited; however, the allergic diseases are preferably accompanied by inflammation. Specific examples of allergic diseases include, but are not limited to, contact dermatitis accompanied by Type IV reaction (delayed allergic reaction) according to Coombs classification; and, although they are not classified into Type IV, allergic rhinitis, allergic asthma, atopic dermatitis, acute disseminated encephalomyelitis, and hay fever accompanied by inflammation.

Among the above diseases, examples of the symptoms accompanying organ transplants include, but are not limited to, symptoms (preferably inflammation) accompanying organ transplants that occur between the organ donors and the organ recipients due to immune response. Specific examples of morbid conditions of the symptoms accompanying organ transplants include, but are not limited to, graft-versus-host disease, and acute and chronic rejection responses.

The target diseases of the present invention include various diseases of various animals. Preferably, the diseases are diseases of human or nonhuman mammals such as apes, mice, rats, canine, rabbits, bovine, and horses; more preferably diseases of human and mice; and particularly preferably diseases of human.

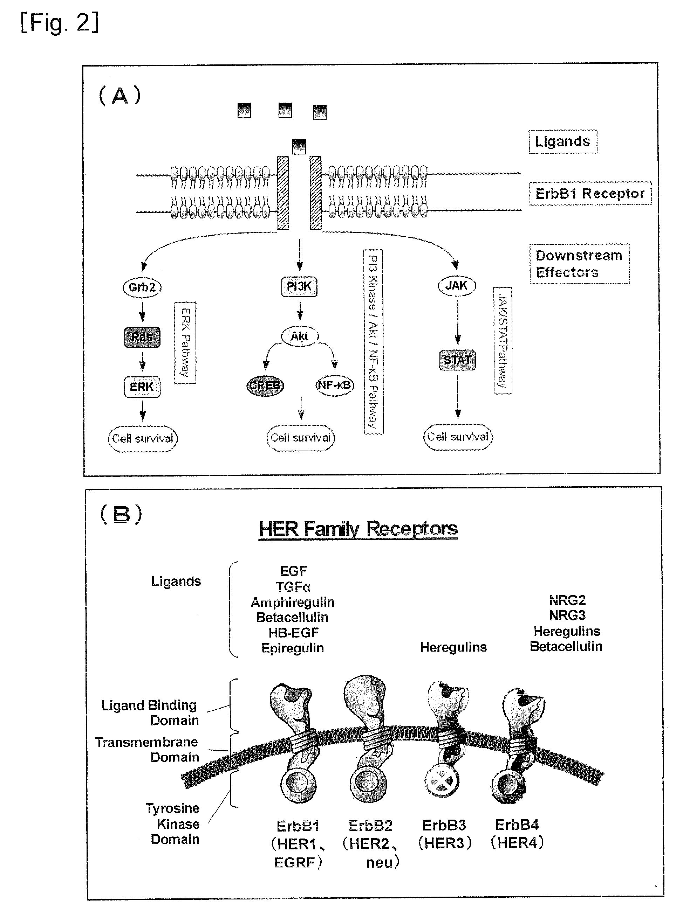

The prophylactic or therapeutic agent of the present invention contains a compound that inhibits expression of the function of a protein belonging to ErbB1 pathway. The ErbB1 pathway is a signal pathway known by persons skilled in the art. The ErbB1 pathway is shown in Fig. 2(A). The aforementioned protein is constituted of ErbB1 protein as a receptor, a ligand of ErbB1 protein, and a downstream effecter.

The receptor is ErbB1 protein (also referred to as HER1 or EGFR). ErbB1 protein is a known tyrosine kinase receptor belonging to HER family. In addition to ErbB1 protein, ErbB2 protein (also referred to as HER2 or Neu), ErbB3 protein (also referred to as HER3) and ErbB4 protein (also referred to as HER4) are known as the proteins of HER family. However, it is known that, as shown in a later-described example, ErbB2 protein is distinguished from ErbB1 protein, and ErbB3 protein and ErbB4 protein do not belong to the ErbB1 pathway.

The ligand is a ligand for ErbB1 protein serving as a receptor. Known examples of the ligand include, but are not limited to, EGF protein, HB-EGF protein, TGF-α protein, epiregulin protein, amphiregulin protein, and betacellulin protein. Preferably, the ligand is selected from EGF protein, HB-EGF protein, and epiregulin protein; more preferably epiregulin protein.

Examples of the downstream effecter include, but are not limited to, proteins belonging to the ERK pathway (such as Grb2 protein, Ras protein, or ERK (MAPK) protein), proteins belonging to the PI3 kinase/Akt/NF-κB pathway (such as phosphatidyl inositol-3 kinase (PI-3 kinase, PI3K), akt protein, GSK3 protein, eIF2B protein, or NF-κB protein), proteins belonging to the JAK/STAT pathway (such as JAK protein or STAT protein). The downstream effecter is preferably a protein belonging to the PI3 kinase/Akt/NF-κB pathway, more preferably PI3 kinase and NF-κB, and particularly preferably PI3 kinase.

PI3 kinase is assumed to be a dimeric protein constituted of a catalyst unit (p110) and a control unit (p85). PI3 kinase can be classified by p110 sub-type, which includes α-type having p110α, β-type having p110β, γ-type having p110γ, and δ-type having p110δ. In the present invention, the PI3 kinase is not particularly limited, but is preferably α-type PI3 kinase.

In the present invention, the protein belonging to ErbB1 pathway is preferably selected from ErbB1 protein and epiregulin protein, particularly preferably epiregulin protein.

The amino acid sequence of the above protein and the base sequence of the gene encoding the protein are publicly known; for example, they are published in the database of the National Institute of Health (NIH). Further, the protein belonging to ErbB1 pathway can be derived from human or nonhuman mammals such as apes, mice, rats, canine, rabbits, bovine, and horses.

The therapeutic or prophylactic agent of the present invention contains a compound that inhibits the expression of the function of a protein belonging to ErbB1 pathway. The therapeutic or prophylactic agent of the present invention may contain one or two or more kinds of the compounds. The compound is not limited within a capability inhibiting the expression of the function of a protein belonging to ErbB1 pathway. Here, the representation "inhibition of the expression of the function of a protein" means various aspects of inhibition of expression of the function of a protein. Examples of the aspects include, but are not limited to, inhibition of the function of a protein, inhibition of the expression of a protein (inhibition of the transcription of a gene encoding a protein, inhibition of the translation of the protein, etc.). Examples of the aspects of inhibition of the function of a protein include, but are not limited to, if the protein is ErbB1 protein, inhibition of the bond of ErbB1 protein with a ligand, inhibition of the dimerization of ErbB1 protein, and inhibition of the enzymatic activity, such as tyrosine kinase activity of ErbB1 protein. If the protein is a ligand of ErbB1 protein, examples of the aspects include, but are not limited to, inhibition of the bond of ErbB1 protein with a ligand. If the protein is a downstream effecter of ErbB1 protein, examples of the aspects include, but are not limited to, inhibition of the bond of the protein and ErbB1 protein or other target proteins; and inhibition of the enzymatic activity, such as kinase activity, of the protein.

Specific examples of the compounds include, but are not limited to, an inhibitor of a protein belonging to ErbB1 pathway, an antibody that specifically binds to a protein belonging to ErbB1 pathway, and a nucleic acid that inhibits the expression of a protein belonging to ErbB1 pathway.

The inhibitor of a protein belonging to ErbB1 pathway may be any inhibitor of a protein belonging to ErbB1 pathway, including existing inhibitors, or inhibitors to be developed in the future. Preferably, the inhibitor is a specific inhibitor of a protein belonging to ErbB1 pathway.

Examples of ErbB1 protein inhibitors include, but are not limited to, a kinase inhibitor for ErbB1 protein. Examples of the kinase inhibitors for ErbB1 protein include known PD153035 (4-[(3-Bromophenyl)amino]-6,7-dimethoxyquinazoline), PD168393 (4-[(3-Bromophenyl)amino]-6-acrylamidoquinazoline), gefitinib gefitinib(N-(3-chloro-4-fluoro-phenyl)-7-methoxy-6-(3-morpholin-4-ylpropoxy) quinazolin-4-amine (product name: Iressa (registered trademark)), and erlotinibe (N-(3-ethynylphenyl)-6,7- bis(2-methoxyethoxy)-4-quinazolinamine (product name: Tarceva (registered trademark)); pharmaceutically acceptable salts thereof; and derivatives thereof having an inhibitory activity against the tyrosine kinase activity of ErbB1 proteins.

Further, other kinase inhibitors for ErbB1 proteins may also be used. Examples thereof include vandetanib (N-(4-bromo-2-fluorophenyl)-6-methoxy-7-[(1-methylpiperidin-4-yl)methoxy]quinazolin-4-amine (product name: Zactima (registered trademark)), lapatinib (n-[3-chloro-4-[(3-fluorophenyl)methoxy]phenyl]-6-[5-[(2-methylsulfonylethylamino)methyl]-2-furyl]quinazolin-4-amine (product name: Tykerb), and ARRY-543(4-N-[3-chloro-4-(thiazol-2-ylmethoxy)phenyl]-6-N-[(4R)-4-methyl-4,5-dihydrooxazol-2-yl]quinazoline-4,6-diamine bis (4-methylbenzenesulfonate) (Array BioPharma Inc.).

Furthermore, inhibitors for ErbB1 proteins other than kinase inhibitors may also be used. Examples thereof include XL647 (also called EXEL-7647, (Exelixis)), PF-00299804 (also called PF299, (Pfizer Inc.)), and TAK-285 (Takeda Pharmaceutical Company Limited).

Among the aforementioned kinase inhibitors for ErbB1 proteins, gefitinib, erlotinib, vandetanib, lapatinib, ARRY-543, XL647, PF-00299804, and TAK-285 are known as anti-cancer drugs.

Examples of the specific inhibitors for downstream effecters of ErbB1 proteins include, but are not limited to, specific kinase inhibitors for PI3 kinase, which is a downstream effecter of ErbB1 protein. Examples of specific kinase inhibitors of PI3 kinase include LY294002 (2-(4-Morpholinyl)-8-phenyl-4H-1-benzopyran-4-one) and PIK75 (2-methyl-5-nitro-2-[(6-bromoimidazo[1,2-a]pyridin-3-yl)methylene]-1-methylhydrazide-benzenesulfonic acid); pharmaceutically acceptable salts thereof; and derivatives thereof having an inhibitory activity against PI3 kinase. Preferably, the specific kinase inhibitors for PI3 kinase are specific kinase inhibitors against PI3 kinase α such as PIK75.

The antibodies that specifically bind to proteins belonging to the ErbB1 pathway may be any antibodies that inhibit the functions of the proteins belonging to the ErbB1 pathway, including existing antibodies, and antibodies to be developed in the future. Preferably, the antibodies are antibodies that bind to the active site of the protein belonging to ErbB1 pathway, and inhibit the function. If the protein is ErbB1 protein serving as a receptor, examples of the active sites include, but are not limited to, the binding site of the ErbB1 protein with the ligand, the enzymatic active center of the tyrosine kinase of the ErbB1 protein, the binding site of the ErbB1 protein with the downstream effecter, and the like. If the protein is the aforementioned ligand (such as epiregulin protein), examples of the active sites include, but are not limited to, the binding site of the ligand with ErbB1 protein. If the protein is the downstream effecter of ErbB1 protein, examples of the active sites include, but are not limited to, the binding site of the downstream effecter with the ErbB1 protein or other target proteins, and the enzymatic active center of, such as kinase activity, of the protein.

The antibodies may be selected from polyclonal antibodies and monoclonal antibodies. The polyclonal antibodies and monoclonal antibodies can be appropriately produced by a person skilled in the art according to a known method. Examples of monoclonal antibodies include chimeric antibodies, humanized antibodies, and human antibodies produced by a known method.

Specific examples of the above antibodies include, but are not limited to, cetuximab (product name: Erbitux) (registered trademark), panitumumab (product name: Vectibix) (registered trademark), zalutumumab (product name: HuMax-EGFr), necitumumab (also called IMC-11F8), nimotuzumab (product name: Theraloc, Vecthix, BiomAb-EGFR), matuzumab (also called EMD 72000), and Ch806 (Life Science Pharmaceuticals Inc.).

Among the aforementioned antibodies, cetuximab, panitumumab, zalutumumab, necitumumab, nimotuzumab, matuzumab, and Ch806 are known as anti-cancer drugs.

When the protein belonging to ErbB1 pathway is epiregulin protein, preferable examples of the antibodies that specifically bind to the proteins belonging to the ErbB1 pathway include antibodies having neutralization activity, such as Cat# MAB1068 antibodies (R&D Systems).

Examples of nucleic acids that inhibit expression of the protein belonging to ErbB1 pathway include RNA molecules that target a gene encoding the protein belonging to ErbB1 pathway and DNA molecules such as vectors enabling expression of the low-molecular-weight RNA. Specific examples of the above RNA molecules include, but are not limited to, RNA molecules having an RNA interference effect (an effect considered to derive from specific destruction of mRNA of the target gene) such as siRNA, shRNA, or dsRNA; and miRNA, which is considered to inhibit the translation of mRNA of the target gene.

The RNA molecules having an RNA interference effect can be appropriately designed by a person skilled in the art according to a known method using the information of the base sequence of the target gene. Further, the RNA molecules can be appropriately produced by a person skilled in the art according to a known method. Commercially available RNA molecules and nucleic acids may also be used. Preferably, the RNA molecules are siRNA, shRNA, or miRNA; particularly preferably siRNA and shRNA.

The miRNA may be, but is not limited to, RNA having an activity of inhibiting transcription or translation of the above gene.

The DNA molecules such as vectors capable of expressing the RNA molecules can be appropriately produced by a person skilled in the art according to a known method. Specific examples of the above vectors include, but are not limited to, adenovirus vector, lentivirus vector, and adeno-associated virus vector. Preferably, the vector is a lentivirus vector.

The therapeutic or prophylactic agent of the present invention is preferably provided as a pharmaceutical composition. The pharmaceutical composition can be processed into a preparation by a known method. Examples of the preparations include, but are not limited to, solid preparations such as tablets, capsules, pills, powdered drug, or granules, and liquid preparations such as liquid agents, suspensions, emulsions, or injections. As required, it is also possible to add a pharmaceutically acceptable carrier and additive agents depending on the preparation form. Specific examples of the above carriers and additive agents include, but are not limited to, excipients, fillers, binders, moisturizers, fragrances, and coloring agents. If the preparation is a liquid preparation, known pharmaceutically acceptable solvents, for example, physiological saline solution, buffer solutions, and the like may be used.

The administration amount of the therapeutic or prophylactic agent of the present invention is not particularly limited, and may be determined by a person skilled in the art to an appropriate amount insofar as the treatment or prophylactic effect with respect to the above diseases can be ensured. The administration amount is, for example, as a single dose, 0.01 to 1000 mg, preferably 0.05 to 500 mg, more preferably 0.1 to 100 mg per 1 kg of the patient's body weight.

The administration method of the therapeutic or prophylactic agent of the present invention is not particularly limited, and an appropriate method may be selected by a person skilled in the art insofar as the treatment or prophylactic effect with respect to the above diseases can be ensured. For example, the person skilled in the art may select an administration method specifically required to treat the morbid conditions of the above diseases. Specific examples of the administration methods include, but are not limited to, administration by injection (intravenous injection, hypodermic injection, intramuscular injection, intraperitoneal injection, injection to the affected part, etc.), oral administration, suppository administration, and dermal administration (such as application).

The present invention further provides a therapeutic method or a prophylactic method that treats or prevents autoimmune diseases, inflammatory diseases, allergic diseases and like diseases, and symptoms accompanying organ transplants. The method comprises a step of administering a compound that inhibits expression of the function of a protein belonging to ErbB1 pathway. The target, method, and amount of the administration are as described above.

The present invention further provides the aforementioned compound that inhibits the function of a protein belonging to ErbB1 pathway; the compound is used for the treatment or prevention of autoimmune diseases, inflammatory diseases, allergic diseases and like diseases, and symptoms accompanying organ transplants.

The present invention further provides use of the compound that inhibits expression of the function of a protein belonging to ErbB1 pathway for the manufacture of a therapeutic or prophylactic agent that treats or prevents autoimmune diseases, inflammatory diseases, allergic diseases and like diseases, and symptoms accompanying organ transplants.

### (2) Immunosuppressant

The compound that inhibits expression of the function of a protein belonging to ErbB1 pathway described in Item (1) above greatly reduces production of inflammatory cytokine such as IL-6, as shown in the later-described Examples and Reference Examples. The reduction of inflammatory cytokine is considered to suppress immune reaction. Therefore, the present invention also provides an immunosuppressant containing the compound that inhibits expression of the function of a protein belonging to ErbB1 pathway.

The immunosuppressant is suitable for patients who need immunosuppression, patients who have excessive immune reaction, and the like. Examples of the patients include, but are not limited to, patients having diseases or symptoms including autoimmune diseases, inflammatory diseases, allergic diseases and like diseases, and symptoms accompanying organ transplants.

The compound described in Item (1) above can be suitably used for the compound that inhibits expression of the function of a protein belonging to ErbB1 pathway.

The immunosuppressant may suitably be provided as the pharmaceutical composition described in Item (1) above.

The immunosuppressant may suitably be used in the administration amount and administration method described in Item (1) above.

### (3) Inflammatory Cytokine Production Inhibitor

As shown in the later-described Examples and Reference Examples, the compound that inhibits expression of the function of a protein belonging to ErbB1 pathway described in Item (1) above greatly reduces production of inflammatory cytokine such as IL-6 protein. Therefore, the present invention also provides an inflammatory cytokine production inhibitor containing the compound that inhibits expression of the function of a protein belonging to ErbB1 pathway.

The inflammatory cytokine includes various known cytokines known as inflammatory cytokines having an activity of promoting inflammatory reaction and immune reaction. Specific examples of inflammatory cytokines include, but are not limited to, TNF-α protein, IFN-γ protein, IL-1 protein, IL-6 protein, IL-8 protein, IL-12 protein, IL-17 protein, and IL-18 protein. The inflammatory cytokine is preferably IL-1 protein, IL-6 protein, IL-17 protein, or TNF-α protein, more preferably IL-6.

The inflammatory cytokine production inhibitor is suitably used as a therapeutic or prophylactic agent for patients who need suppression in the production of inflammatory cytokine, patients who have excessive inflammatory cytokine production, and the like. Examples of the patients include, but are not limited to, patients having diseases or symptoms including autoimmune diseases, inflammatory diseases, allergic diseases and like diseases, or symptom accompanying organ transplants.

The compound described in Item (1) above can be suitably used for the compound that inhibits expression of the function of a protein belonging to ErbB1 pathway.

The inflammatory cytokine production inhibitor may suitably be provided as the pharmaceutical composition described in Item (1) above.

The inflammatory cytokine production inhibitor may suitably be used in the administration amount and administration method described in Item (1) above.

### (4)IL-6 Amplifier Inhibitor

As shown in the later-described Examples and Reference Examples, the compound that inhibits expression of the function of a protein belonging to ErbB1 pathway described in Item (1) above induces to decrease chemokine production, such as IL-6 and CCL20 proteins, KC protein, Mip2 protein, CXCL5 protein, or CCL2 protein. The decrease is supposed to due to significant suppression in the activity of IL-6 amplifier. Therefore, the present invention also provides an IL-6 amplifier inhibitor containing the compound that inhibits expression of the function of a protein belonging to ErbB1 pathway.

Here, "IL-6 amplifier" designates an IL-17A-triggered amplification loop (positive feedback loop) (shown in Fig. 1) contributing the production of inflammatory cytokine found in Non-patent Document 1. The constituents of IL-6 amplifier include IL-6 protein, IL-17A protein, IL-7 protein, and like cytokines; and NF-κB, STAT3 protein, and like transcription factors. The action mechanism is considered to be based on a positive feedback loop in which IL-17A protein produced by Th-17 cells and IL-6 protein cooperate to accelerate the activities of NF-κB and STAT3 protein in fibroblasts (type I collagen-positive cells); the acceleration in the activities of NF-κB and STAT3 protein induces accelerating the production of inflammatory cytokine such as IL-6 protein or chemokine; and the accelerating the production of inflammatory cytokine results in more production of IL-6 protein and IL-17A protein.

The IL-6 amplifier inhibitor is suitably used as a therapeutic or prophylactic agent for patients who need suppression in IL-6 amplifier activity, patients who have excessive IL-6 amplifier activity, and the like. Examples of the patients include, but are not limited to, patients having diseases or symptoms including autoimmune diseases, inflammatory diseases, allergic diseases and like diseases, and symptoms accompanying organ transplants. The IL-6 amplifier inhibitor may also be used as a tool for the study of IL-6 amplifier, or for technical development in a field related to IL-6 amplifier.

The compound described in Item (1) above can be suitably used as the compound that inhibits expression of the function of a protein belonging to ErbB1 pathway.

To use the IL-6 amplifier inhibitor as a medicament, the IL-6 amplifier inhibitor may suitably be provided as the pharmaceutical composition described in Item (1) above.

To use the IL-6 amplifier inhibitor as a medicament, the IL-6 amplifier inhibitor may suitably be used in the administration amount and administration method described in Item (1) above.

### (5) Screening Method

The present invention relates to a method for screening, among test substances, an active ingredient of a therapeutic or prophylactic agent for at least one disease or symptom selected from the group consisting of autoimmune diseases, inflammatory diseases, allergic diseases and symptoms accompanying organ transplants. The diseases and symptoms are preferably the diseases and symptoms described in Item (1) above. The therapeutic or prophylactic agent may be a therapeutic or prophylactic pharmaceutical composition containing the active ingredient for treating the above diseases, and the like.

The screening method of the present invention comprises the steps of: (i) determining whether a test substance is a compound that inhibits expression of the function of a protein belonging to ErbB1 pathway, and (ii) selecting the test substance determined as a compound that inhibits expression of the function of a protein belonging to ErbB1 pathway in Step (i) as an active ingredient of the therapeutic or prophylactic agent.

Step (i) determines whether a test substance subjected to the screening is a compound that inhibits expression of the function of a protein belonging to ErbB1 pathway.

The test substance is not particularly limited insofar as it is an appropriate candidate for an active ingredient of the therapeutic or prophylactic agent for the above diseases and the like. The test substance may be either natural compounds (for example, substances derived from organisms) or synthetic compounds. Preferably, the test substance is a compound that is pharmaceutically acceptable in human. Specific examples of the test substances include, but are not limited to, low-molecular-weight compounds, proteins such as antibodies, nucleic acids that suppress expression of protein (for example, siRNA, shRNA, dsRNA, miRNA, etc.), sugar chains, and complex carbohydrates.

The protein belonging to ErbB1 pathway is preferably the protein described in Item (1) above.

The means for determining the compound that inhibits expression of the function of a protein belonging to ErbB1 pathway may be appropriately selected by a person skilled in the art from various means including existing means, or means to be developed in the future, depending on the target test substance and the expression of the function of the protein belonging to ErbB1 pathway in which the protein is to be examined for suppression insofar as the objective is achieved.

Here, the expression "inhibit expression of the function of a protein" generally means, but is not limited to, various aspects of inhibition of expression of the function of a protein. Examples of the aspects include, but are not limited to, inhibition of the function of a protein, inhibition of the expression of a protein (inhibition of the transcription of a gene encoding a protein, inhibition of the translation of the protein, etc.). Examples of the aspects of inhibition of the function of a protein include, but are not limited to, if the protein is ErbB1 protein, inhibition of the bond of ErbB1 protein with a ligand, inhibition of the dimerization of ErbB1 protein, and inhibition of the enzymatic activity, such as tyrosine kinase activity of ErbB1 protein. If the protein is a ligand of ErbB1 protein, examples of the aspects include, but are not limited to, inhibition of the bond of ErbB1 protein with a ligand. If the protein is a downstream effecter of ErbB1 protein, examples of the aspects include, but are not limited to, inhibition of the bond of the protein with ErbB1 protein or other target proteins; and inhibition of the enzymatic activity, such as kinase activity, of the protein.

As required, the test substance determined as the compound that inhibits expression of the function of a protein belonging to ErbB1 pathway in Step (i) may further be determined as to whether the compound can suppress the expression of inflammatory cytokine such as IL-6 protein (for example, suppression of transcription of a gene encoding the inflammatory cytokine, suppression of translation of the inflammatory cytokine, etc.). As is evident from the above section "Background Art," etc., this is because a disease accompanied by inflammation is assumed to be caused by excessive production of inflammatory cytokine is considered to be effectively treated or prevented by using a therapeutic or prophylactic agent containing an active ingredient that effectively suppresses expression of the inflammatory cytokine.

The means for determining the compound that inhibits the expression of the inflammatory cytokine may be appropriately selected by a person skilled in the art from various means, including existing means, or means to be developed in the future insofar as the objective is achieved.

As the means for determining the compound that inhibits the expression of the inflammatory cytokine, the following means including Steps (a) and (b) may be used. However, the means are not limited to the following means.
Step (a) of comparing expression amount of inflammatory cytokine between a case of applying the test substance determined as a compound that inhibits expression of the function of a protein belonging to ErbB1 pathway in Step (i) and a case of a control under a condition of activating of IL-6 amplifier in a cell which the IL-6 amplifier works (for example, mouse type I collagen-positive cells BC-1, mouse embryonic fibroblast (MEF), etc.); and
Step (b) of determining the test substance as a compound that significantly suppresses expression of inflammatory cytokine if the above comparison reveals that the test substance significantly suppresses expression of inflammatory cytokine (for example, p value is about <0.05 or <0.01 according to the t-test) compared with the control group.

The activation of IL-6 amplifier can be accomplished, for example, by incorporating IL-6 protein and IL-17A protein in a solution (preferably a culture medium solution, more preferably a serum-free culture medium such as RPMI 1640 culture medium) containing the cells. The activation of IL-6 amplifier can also be performed, for example, by further incorporating epiregulin protein as in the later-described Reference Examples. However, as shown in the later-described Reference Examples, the activation of IL-6 amplifier is considered to increase the expression of epiregulin gene; therefore, in view of accurately determining a compound that suppress expression of inflammatory cytokine, it is preferable that the solution containing the cells is epiregulin protein-free. As demonstrated in the later-described Examples, the determination of a compound that suppresses expression of inflammatory cytokine was successful when the IL-6 amplifier was activated in a culture medium solution that does not contain epiregulin protein.

In Step (ii), an active ingredient of the therapeutic or prophylactic agent for the above diseases or the like is selected. Step (ii) may be performed by selecting the test substance determined as a compound that inhibits expression of the function of a protein belonging to ErbB1 pathway, and that suppresses expression of inflammatory cytokine.

### [Examples]

The present invention is described below in more detail with reference to Reference Examples and Examples. However, the scope of the invention is not limited to these Reference Examples and Examples.

### Reference Example 1

Candidates for causative factors that activate IL-6 amplifier synergistically with STAT3 protein and NF-κB protein to cause IL-6 production were screened.

### Experimental Method

The MISSION (registered trademark) Whole Viral Library (the RNAi Consortium), which is a library of lentiviruses carrying shRNA, was introduced into mouse type I collagen-positive cells BC-1 in accordance with the manual attached thereto, and cells into which lentiviruses carrying shRNA were introduced were selected by puromycin selection.

The obtained clones were cultured for 24 hours in 96-well plates in an RPMI 1640 medium in the presence of 10% fetal bovine serum (FBS). The RPMI 1640 medium contained 50 ng/ml of human IL-6 (hIL-6) protein (Toray), 50 ng/ml of human soluble IL-6 receptor ((hIL-6R protein, R&D Systems, Inc.), and 50 ng/ml of mouse IL-17A protein (mIL-17A, R&D Systems, Inc.).

After the culture, the production amount of mouse IL-6 (mIL-6) protein in each of the clones, i.e., the amount of mIL-6 protein in the medium solution, was quantified by an ELISA method. In the ELISA method, anti-mIL-6 antibody and an ELISA kit, both manufactured by eBioscience, Inc., were used. In addition, a cell proliferation assay was performed for each of the clones, using a TetraColor ONE kit (Seikagaku Corporation).

Selected as candidates for genes that activate IL-6 amplifier synergistically with STAT3 protein and NF-κB protein to cause IL-6 protein production were genes targeted by lentiviruses carrying shRNA introduced into clones in which the production amount of mIL-6 protein in the infected clone cells was 25% or less of the average of those of all clones in the 96-well plates, and in which the results of the cell proliferation assay exhibited 90% or more of the average of those of all clones.

### Experimental Results

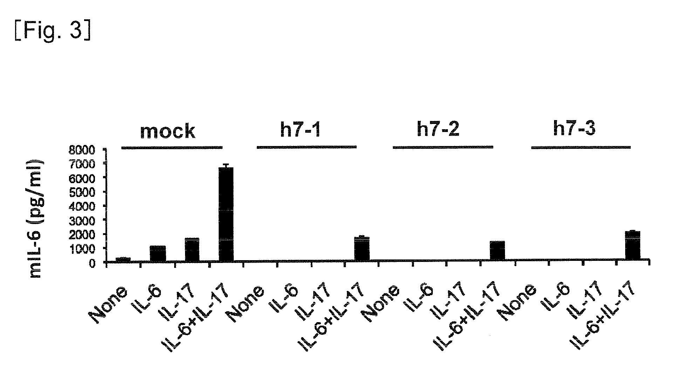

The selected candidate genes included ErbB1 gene and epiregulin gene, in addition to genes involved in IL-6 signal transduction system (such as gp130, JAK, and STAT3) and genes involved in IL-17 signal transduction system (such as IL-17 receptor, TRAF6, and NF-κB). Fig. 3 shows the results of an additional test for shRNA clones that target ErbB1 gene. Culture was performed in the presence of puromycin under each of conditions (e) to (h) of Reference Example 2 described below. When the expression of ErbB1 gene was suppressed by shRNA, the expression amount of mIL6 protein was decreased as compared to the control, in which the expression of ErbB1 gene was not suppressed by shRNA.

### Reference Example 2

The effect of the presence or absence of serum in IL-6 protein production by the activation of IL-6 amplifier was verified.

### Experimental Method

2×10⁵ mouse BC-1 cells were cultured in an RPMI 1640 medium for 24 hours under each of the following conditions:
(a) in the presence of 10% FBS,
(b) with the addition of 50 ng/ml of hIL-6 protein and 50 ng/ml of hIL-6R protein (hereinafter referred to as "hIL-6/6R proteins") and in the presence of 10% FBS,
(c) with the addition of 50 ng/ml of mIL-17A protein and in the presence of 10% FBS,
(d) with the addition of hIL-6/6R proteins at 50 ng/ml and mIL-17A protein at 50 ng/ml and in the presence of 10% FBS,
(e) in the absence of FBS,
(f) with the addition of hIL-6/6R proteins each at 50 ng/ml and in the absence of FBS,
(g) with the addition of 50 ng/ml of mIL-17A protein and in the absence of FBS, or
(h) with the addition of hIL-6/6R proteins each at 50 ng/ml and mIL-17A protein at 50 ng/ml and in the absence of FBS.

After the culture, the production amount of mIL-6 protein in each of the clones was quantified by an ELISA method in the same manner as in Reference Example 1.

In addition, the expression amounts of mIL-6 gene were quantified by a real-time RT-PCR method. The real-time RT-PCR method was performed using a GeneAmp 5700 sequence detection system (Applied Biosystems) and a SYBER green PCR Master Mix (Sigma-Aldrich Co. LLC.). The conditions of the PCR amplification were as follows: the amplification was performed in 40 cycles, each cycle consisting of thermal denaturation (94°C, 15 seconds), annealing (60°C, 30 seconds), and extension reaction (72°C, 30 seconds)).

As primers in the real-time RT-PCR method, primer sets having the following base sequences were used.
mIL-6 gene
   Forward primer: 5'-GTGGCAGGTAGAGCAGGAAG-3' (SEQ ID NO: 1)
   Reverse primer: 5'-CCACCTGAAAGGCACTCTGT-3' (SEQ ID NO: 2)
Mouse HPRT gene
   Forward primer: 5'-GATTAGCGATGATGAACCAGGTT-3' (SEQ ID NO: 3)
   Reverse primer: 5'-CCTCCCATCTCCTTCATGACA-3' (SEQ ID NO: 4)

Further, a cell proliferation assay was performed for each of the clones, using a TetraColor ONE kit (Seikagaku Corporation).

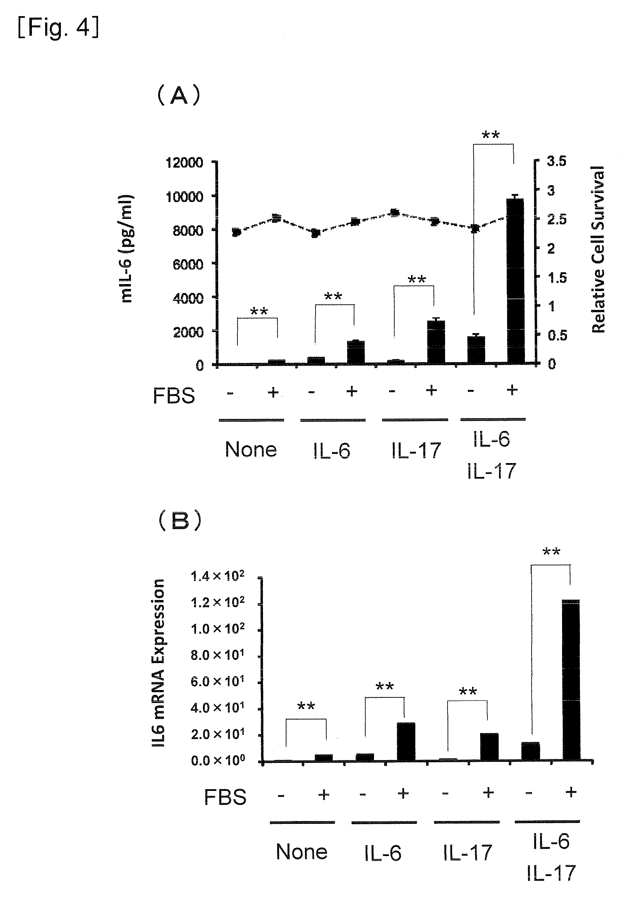

Fig. 4 shows the results. The expression amounts of mIL-6 gene are indicated by relative ratios with respect to the expression amounts of HPRT gene used as a control. Under the conditions in which FBS was added, the expression amounts of mIL-6 protein and the expression amounts of mIL-6 gene were significantly (p value < 0.01) increased.

### Reference Example 3

The effects of the addition of specific growth factors in IL-6 production by the activation of IL-6 amplifier were verified.

### Experimental Method

2×10⁵ mouse BC-1 cells were cultured for 24 hours in an RPMI 1640 medium containing hIL-6/6R proteins each at 50 ng/ml and mIL-17A protein at 50 ng/ml under the condition in which 100 ng/ml of mouse EGF protein (R&D Systems, Inc.), 100 ng/ml of human HB-EGF protein (R&D Systems, Inc.), 0.1, 1, 10, or 100 ng/ml of mouse epiregulin protein (R&D Systems, Inc.), 50 ng/ml of mouse VEGF protein (R&D Systems, Inc.), 50 ng/ml of mouse FGF7 protein (R&D Systems, Inc.), or 300 ng/ml of mPDGF-CC protein (R&D Systems, Inc.) was separately added. Mouse BC-1 cells cultured under the condition in which only hIL-6/6R proteins and mIL-17A protein were added served as a control group.

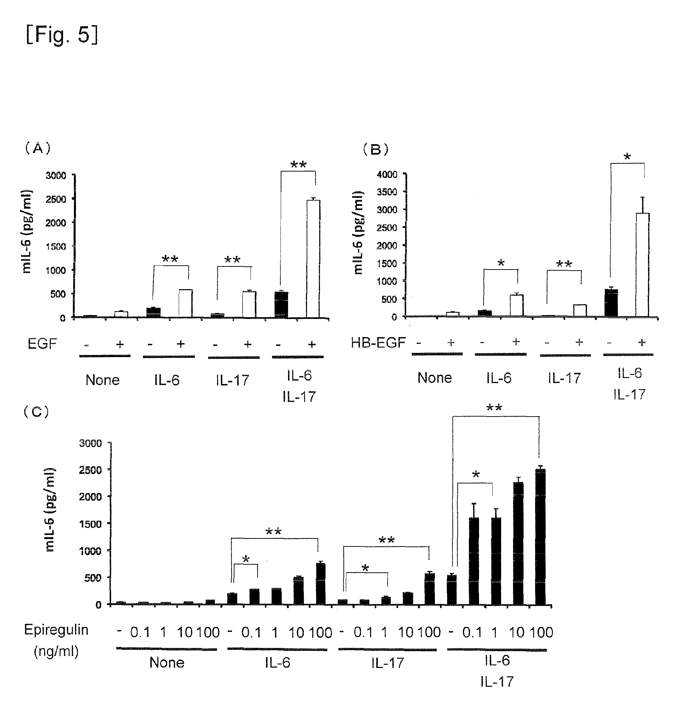

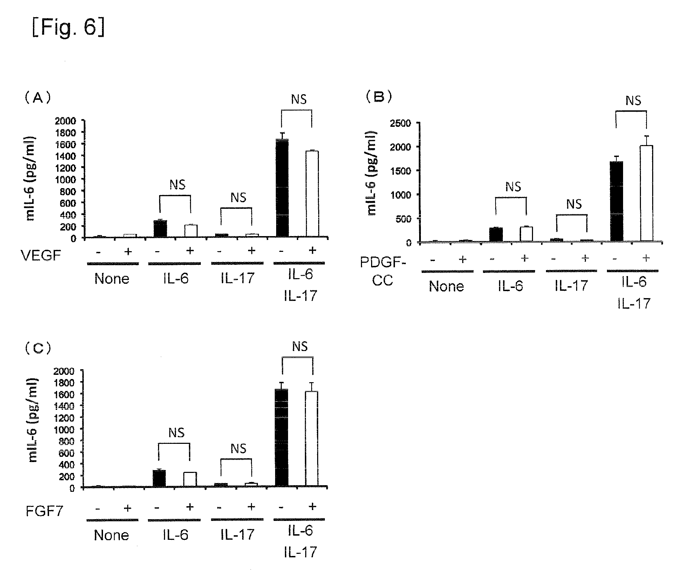

After the culture, the production amount of mIL-6 protein under each condition was quantified by an ELISA method in the same manner as in Reference Example 1. Figs. 5 and 6 show the results. As shown in Fig. 5, under each condition in which EGF protein, HB-EGF protein, or epiregulin protein, which are ligands of ErbB1 protein, was added, the production amount of mIL-6 protein was significantly (p value < 0.01) increased as compared to the case in which none of them was added. In contrast, under each condition in which VEGF protein, FGF7 protein, or PDGF-CC protein, which are not ligands of ErbB1 protein, was added, no significant difference in the production amount of mIL-6 protein from the case in which none of them was added was observed.

### Reference Example 4

The effect of the addition of epiregulin protein on the expression amounts of target genes in IL-6 amplifier was verified.

### Experimental Method

2×10⁵ mouse BC-1 cells or 2×10⁵ mouse embryonic fibroblasts (MEFs) were cultured for 24 hours under each of the conditions (e) to (h) in Reference Example 2, or under each of the conditions (e) to (h) in Reference Example 2 with the addition of 100 ng/ml of mouse epiregulin protein in each of the conditions.

After the culture, the expression amount of mIL-6 gene and the expression amount of mouse CCL20 gene, a target of transcription factor NF-κB, under each condition were quantified by real-time RT-PCR method in the same manner as in Reference Example 2.

As primers in the real-time RT-PCR method, the primer set described in Reference Example 2 and a primer set having the following base sequences were used.
Mouse CCL20 gene
   Forward primer: 5'- ACAGTGTGGGAAGCAAGTCC-3' (SEQ ID NO: 5)
   Reverse primer: 5'- CCGTGAACTCCTTTGACCAT-3' (SEQ ID NO: 6)

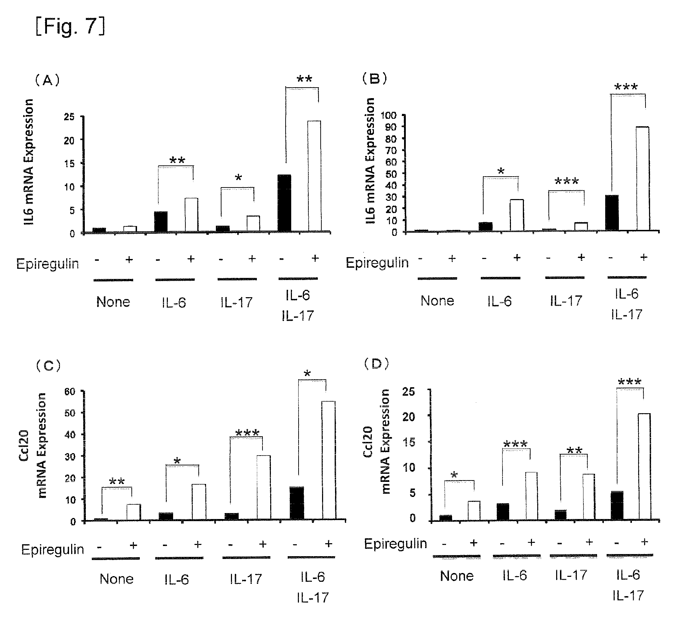

Fig. 7 shows the results. The condition in which no protein was added (condition (e) in Reference Example 2) was used as a control. The expression amounts were indicated by relative ratios with respect to the expression amount of CCL20 gene under the condition (e). In both the BC-1 cells and the MEFs, the amounts of transcription of mIL6 gene were significantly increased by the addition of epiregulin protein.

### Reference Example 5

Specificity of receptors that functions downstream of epiregulin in IL-6 protein production by the activation of IL-6 amplifier was verified.

### Experimental Method

2×10⁵ mouse BC-1 cells were cultured for 24 hours under each of the conditions (e) to (h) of Reference Example 2 with the addition of 100 ng/ml of mouse epiregulin protein in each of the conditions, in the presence of 0, 0.1, 1, or 10 micromoles/L of PD153065 (Calbiochem); or in the presence of 0, 0.1, 1, or 10 micromoles/L of ErbB2 Inhibitor II (Calbiochem, Cat# 324732).

After the culture, the production amount of mouse IL-6 protein under each condition was quantified by an ELISA method in the same manner as in Reference Example 1. Fig. 8 shows the results. Increase in the amount of transcription of mIL-6 due to epiregulin protein was inhibited in a manner dependent on the addition amount of PD153065, an ErbB1 protein inhibitor. In contrast, increase in the amount of transcription of mIL-6 due to epiregulin protein was not inhibited by the addition of ErbB2 Inhibitor II, an ErbB2 protein inhibitor.

### Reference Example 6

The effect of the activation of IL-6 amplifier on the expression amount of epiregulin gene was verified.

### Experimental Method

2×10⁵ mouse BC-1 cells or 2×10⁵ mouse embryonic fibroblasts (MEFs) were cultured for 2 hours, under each of the conditions (e) to (h) of Reference Example 2, or in an RPMI 1640 medium containing 100 ng/ml of mouse epiregulin protein.

After the culture, the expression amount of mouse epiregulin gene under each condition was quantified by a real-time RT-PCR method in the same manner as in Reference Example 2.

As primers in the real-time RT-PCR method, a primer set having the following base sequences was used.
Mouse epiregulin gene
   Forward primer: 5'-CTGCCTCTTGGGTCTTGACG-3' (SEQ ID NO: 7)
   Reverse primer: 5'-GCGGTACAGTTATCCTCGGATTC-3' (SEQ ID NO: 8)

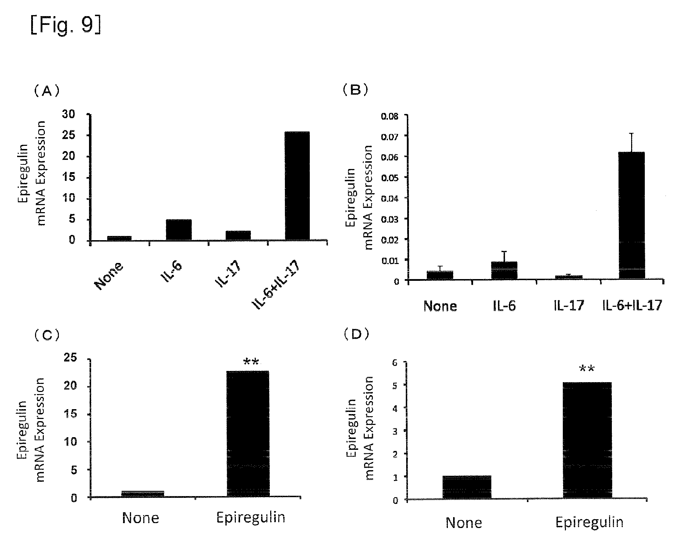

Fig. 9 shows the results. The condition in which no protein was added (condition (e) of Reference Example 2) was used as a control. The expression amounts of mouse epiregulin gene were indicated by relative ratios with respect to the expression amount under the condition (e). In both the BC-1 cells and the MEFs, the addition of hIL-6/6R proteins and mIL-17A protein and the addition of epiregulin protein each increased the amount of transcription of epiregulin gene.

### Reference Example 7

Involvement of epiregulin protein in IL-6 amplifier in synovial cells derived from a human rheumatoid arthritis patient was verified.

### Experimental Method

Synovial cells 050127 derived from a human rheumatoid arthritis patient (Non-Patent Literature 4) were cultured for 3 hours under each of the conditions (e) to (h) of Reference Example 2, or under each of the conditions (e) to (h) of Reference Example 2 with the addition of 100 ng/ml of human epiregulin protein in each of the conditions.

After the culture, the expression amounts of hIL-6 gene were quantified by a real-time RT-PCR method in the same manner as in Reference Example 2.

As primers in the real-time RT-PCR method, a primer set having the following base sequences was used.
Human IL-6 gene
   Forward primer: 5'-GGAGACTTGCCTGGTGAAAA-3' (SEQ ID NO: 10)
   Reverse primer: 5'- GTCAGGGGTGGTTATTGCAT-3' (SEQ ID NO: 11)

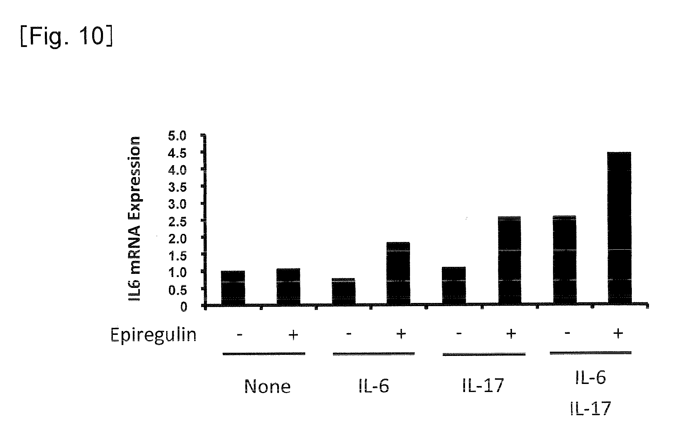

The condition in which no protein was added (condition (e) of Reference Example 2) was used as a control. The expression amounts are indicated by relative ratios with respect to the expression amount under the condition (e). Fig. 10 shows the results. In the synovial cells derived from a human rheumatoid arthritis patient, the amount of transcription of hIL-6 gene was increased by the addition of epiregulin protein in the activation of IL-6 amplifier.

### Reference Example 8

Involvement of abnormality of IL-6 amplifier in the development of dermatitis was verified using disease model mice.

### Experimental Method

Mice of each genotype shown in the following Table 1 were made by a ordinary method by crossing F759 mice (Non-Patent Literature 3) with STAT3^{fl/fl}-K5 Cre mice (Non-Patent Literature 5).

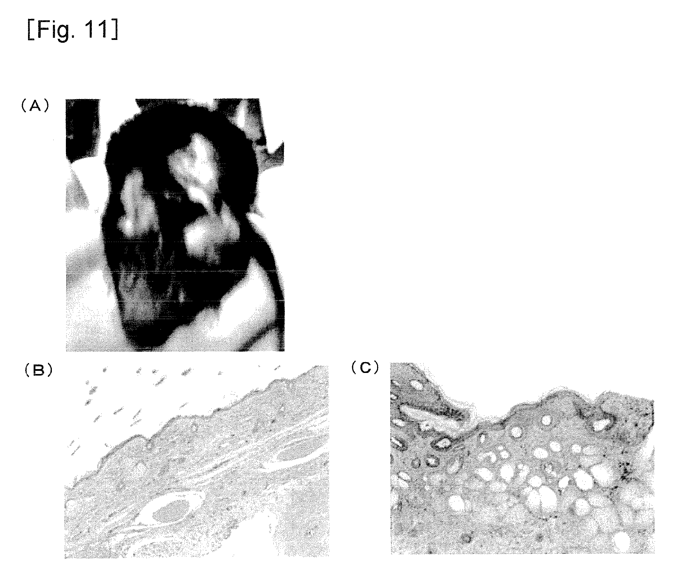

Table 1 shows the incidence of dermatitis, the time of onset, and the number of mice that died within 6 months of age, in the mice of each genotype. Fig. 11 (A) shows an observation image of appearance of a F759/F759-STAT3^{fl/fl}-R5 Cre mouse. Figs. 11 (B) and (C) respectively show hematoxylin-eosin (HE) stained images of skin tissues of a F759/F759-STAT3^{fl/fl}-K5 Cre mouse and of a F759/F759-STAT3^{fl/fl} mouse. In F759/F759-STAT3^{fl/fl}-K5 Cre mice, hair loss believed to be caused by dermatitis was observed, and an image of inflammation with cellular infiltration was also observed in observation of skin tissue sections.

**Table 1**

| Genotype | Incidence (mice) | Time of onset | Death within 6 months of age (mice) |
|---|---|---|---|
| F759/F759-STAT3^{fl/fl}-K5 Cre | 5/5 | 3-8 weeks of age | 5 |
| F759/F759-STAT3^{fl/fl} | 0/5 | - | 0 |
| F759/F759-STAT3^{fl/+}-K5 Cre | 0/7 | - | 0 |
| F759/+-STAT3^{fl/+}-K5 Cre | 5/10 | 4-10 months of age | 0 |
| F759/+-STAT3^{fl/fl} | 0/10 | - | 0 |
| F759/+-STAT3^{fl/+}-K5 Cre | 0/12 | - | 0 |
| +/+-STAT3^{fl/+}-K5 Cre | 3/12 | 4-10 months of age | 0 |

STAT3^{fl/fl}-K5 Cre mice are genetically modified mice in which the STAT3 locus is flanked by loxP sites, Cre recombinase target sites; and in which Cre recombinase expressed by keratin 5 (K5) promoter, a skin tissue-specific promoter, is introduced. Specifically, it is believed that in STAT3^{fl/fl}-K5 Cre mice, skin tissues lack STAT3 gene by the action of Cre recombinase, which is expressed in a skin tissue-specific manner. It is known that about one year after birth, dermatitis develops in STAT3^{fi/fl}-K5 Cre mice due to abnormalities in skin wound healing (Non-Patent Literature 5).

As described below, F759 mice are believed to be mice in which dysregulation of IL-6 amplifier is caused.

Specifically, it was indicated that atopic dermatitis-like symptoms worsen in mice having F756 mutation believed to cause dysregulation of IL-6 amplifier.

### Reference Example 9

Involvement of epiregulin protein in IL-6 amplifier in cells derived from human was verified.

### Experimental Method

1×10⁴ synovial fibroblasts derived from human were cultured for 3 hours under each of the conditions (e) to (h) of Reference Example 2, or under each of the conditions (e) to (h) of Reference Example 2 with the addition of 100 ng/ml of human epiregulin protein in each of the conditions.

After the culture, the expression amounts of hIL-6 gene were quantified by a real-time RT-PCR method in the same manner as in Reference Example 2.

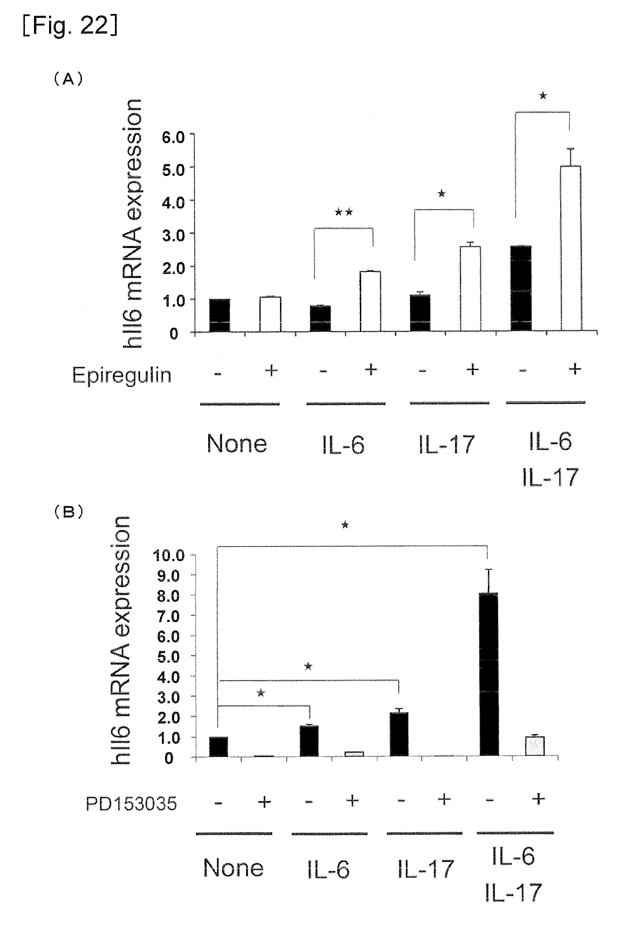

The condition in which no protein was added (condition (e) of Reference Example 2) was used as a control. The expression amounts are indicated by relative ratios with respect to the expression amount under the condition (e). Fig. 22 (A) shows the results. In the activation of IL-6 amplifier, the amount of transcription of hIL-6 gene was increased by the addition of epiregulin protein in the synovial fibroblasts derived from human.

### Reference Example 10

The effect of an ErbB1 protein inhibitor on IL-6 amplifier in cells derived from human was evaluated.

### Experimental Method

1×10⁴ human synovial fibroblasts were cultured for 3 hours in the presence of 0 or 10 micromoles/L of PD153065 (Calbiochem) under each of the conditions (e) to (h) of Reference Example 2 with the addition of 100 ng/ml of mouse epiregulin protein in each of the conditions.

After the culture, the expression amount of human IL-6 gene under each condition was quantified by a RT-PCR method in the same manner as in Reference Example 9. Fig. 22 (B) shows the results. Increase in the amount of transcription of hIL-6 due to epiregulin protein was inhibited in a manner dependent on the addition amount of PD153065, an ErbB1 protein inhibitor.

### Reference Example 11

The effect of the activation of IL-6 amplifier on the expression amount of epiregulin gene in cells derived from human was verified.

### Experimental Method

1×10⁴ synovial fibroblasts derived from human were cultured for 3 hours, under each of the conditions (e) to (h) of Reference Example 2, or in an RPMI 1640 medium containing 100 ng/ml of human epiregulin protein.

After the culture, the expression amount of human epiregulin gene under each condition was quantified by a real-time RT-PCR method in the same manner as in Reference Example 2.

As primers in the real-time RT-PCR method, a primer set having the following base sequences was used.
Human epiregulin gene
   Forward primer: 5'- CTGCCTGGGTTTCCATCTTCT-3' (SEQ ID NO: 12)
   Reverse primer: 5'- GCCATTCATGTCAGAGCTACACT-3' (SEQ ID NO: 13)

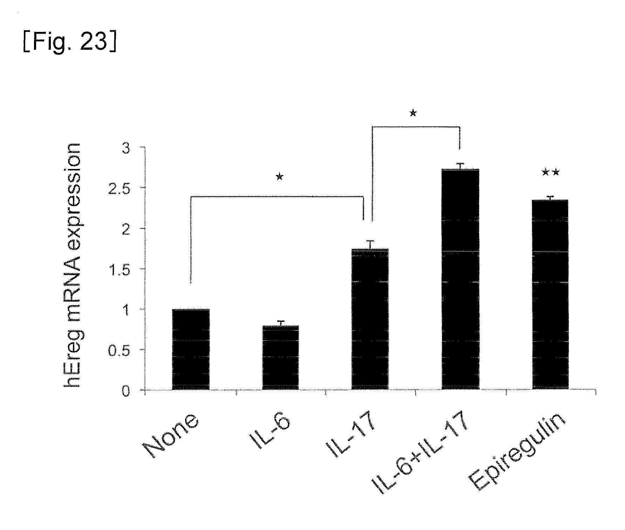

Fig. 23 shows the results. The condition in which no protein was added (condition (e) of Reference Example 2) was used as a control. The expression amounts of mouse epiregulin gene are indicated by relative ratios with respect to the expression amount under the condition (e). The addition of hIL-6/6R proteins and mIL-17A protein and the addition of epiregulin protein each increased the amount of transcription of human epiregulin gene.

### Example 1

The effects of ErbB1 protein inhibitors on IL-6 amplifier in cultured cells were evaluated.

### Experimental Method

2×10⁵ mouse BC-1 cells or 2×10⁵ mouse embryonic fibroblasts (MEFs) were cultured for 30 minutes in an RPMI 1640 medium, in the presence of 0.1, 1, or 10 micromoles/L of PD153065; or in the presence of 0.1, 1, or 10 micromoles/L of PD168393 (Calbiochem).

Subsequently, the cells and the fibroblasts treated with each of the inhibitors were stimulated under each of the conditions (a) to (d) of Reference Example 2, and cultured for 24 hours.

A sample obtained by treating BC-1 cells with an FGF RTK inhibitor (Calbiochem, Cat# 341608) instead of the ErbB1 protein inhibitors and a sample obtained by treating BC-1 cells with an FGF/PDGF/VEGF RTK inhibitor (Calbiochem, Cat# 341610) instead of the ErbB1 protein inhibitors served as control groups.

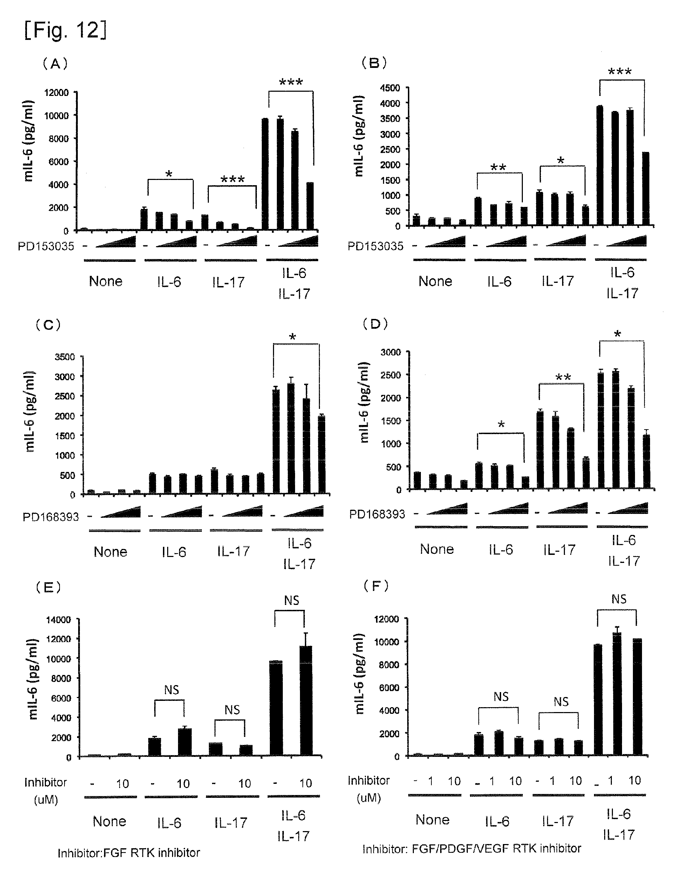

After the culture, the production amount of mouse IL-6 protein under each condition was quantified by an ELISA method. Fig. 12 shows the results. By the addition of the ErbB1 protein inhibitors, increase in the expression amount of mIL-6 protein due to activation of IL-6 amplifier was significantly suppressed in a manner dependent on the addition amount of the inhibitors. In contrast, increase in the expression amount of mIL-6 protein due to activation of IL-6 amplifier was not suppressed by the addition of the inhibitors of receptor-type tyrosine kinases different from ErB1 protein.

### Example 2

The effects of PI3 kinase inhibitors on IL-6 amplifier in cultured cells were evaluated.

### Experimental Method

2×10⁵ mouse BC-1 cells was cultured for 24 hours under each of the conditions (a) to (d) of Reference Example 2 with the addition of 0 or 100 ng/ml of mouse epiregulin protein in each of the conditions, in the presence of 0.1, 1, or 10 micromoles/L of LY294002 (Cell Signaling Technology, Inc.); or in the presence of 0.01, 0.03 (only the case in which mouse epiregulin protein was added), 0.1, 0.3, or 1.0 micromole/L of PIK75 (Cayman Chemical Company).

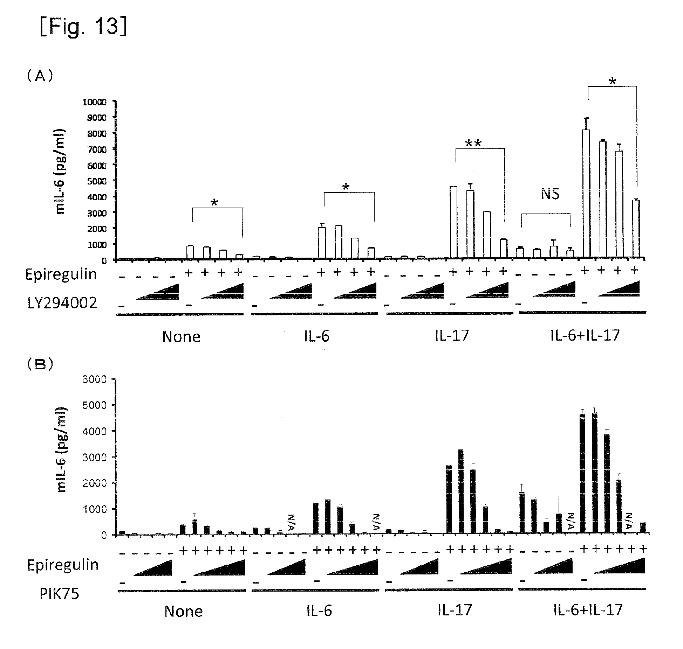

After the culture, the production amount of mouse IL-6 protein under each condition was quantified by an ELISA method. Fig. 13 shows the results. By the addition of the PI3 kinase inhibitors, increase in the expression amount of mIL-6 protein due to the activation of IL-6 amplifier was suppressed in a manner dependent on the addition amount of the inhibitors.

### Example 3

The effect of administration of an ErbB1 protein inhibitor on lesion part in cytokine-induced arthritis mice (disease model mice with rheumatoid arthritis) was evaluated.

### Experimental Method

On each of days 0, 1, and 2, 100 ng of mIL-6 protein and 100 ng of mIL-17A protein, as cytokines, were injected into the malleolus joints of 8-week-old F759 mice to induce arthritis. At the same time, on each of days 0 to 23, 10 micrograms of PD153035 dissolved in DMSO was injected into the malleolus joints (n=6). Mice injected with the cytokines and DMSO only (n=6), and mice injected with DMSO only (n=6) served as control groups.

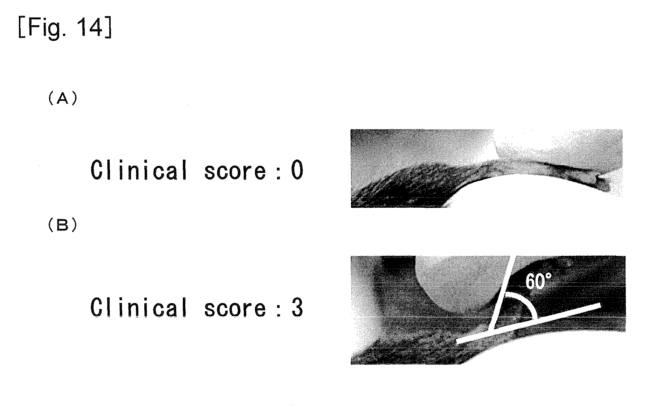

From day 0 to day 23, symptom of arthritis was evaluated by clinical scores. Specific examples of the evaluation are described below and in Fig. 2. Specifically, for example, the clinical score is 0 if the movable range of malleolus joint is a healthy state as shown in Fig. 14 (A), and the clinical score is 3 if severe arthritis develops and the movable range of malleolus joint is 60° smaller than the healthy case as shown in Fig. 14 (B). Further, the clinical score is 1 if the movable range of malleolus joint is 20° smaller than the healthy case, and the clinical score is 2 if the movable range of malleolus joint is 40° smaller than the healthy case.

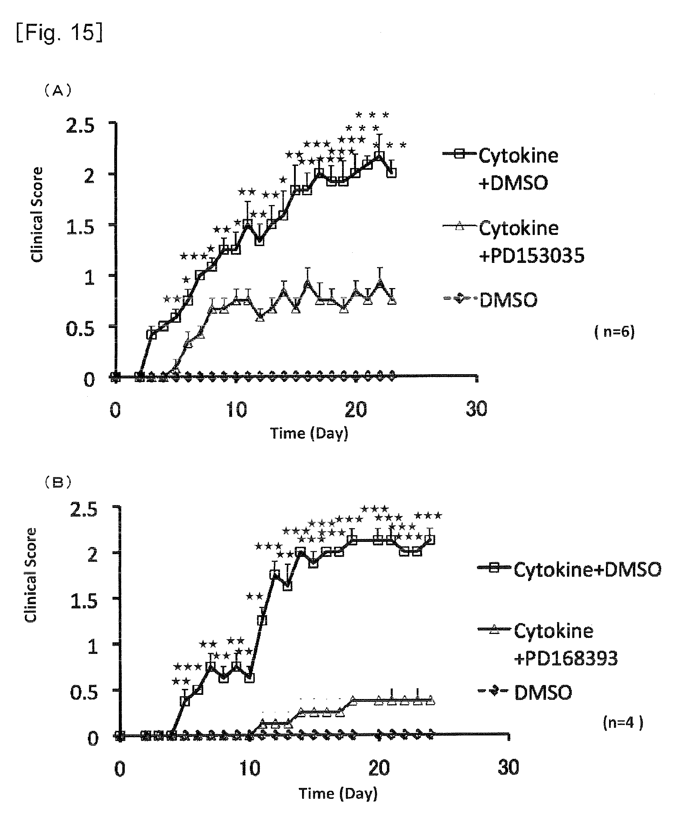

Fig. 15 (A) shows the evaluation results. It was observed that symptom of cytokine-induced arthritis was alleviated, or worsening of symptom of cytokine-induced arthritis was suppressed by administration of PD153035, an ErbB1 protein inhibitor.

F759 mice used as cytokine-induced arthritis mice are genetically modified mice in which F759 mutation (in which tyrosine residue 759 (Y) is replaced by phenylalanine (F) in gp130 protein, a receptor of IL-6 protein, etc.) is introduced (Non-Patent Literature 3); and in F759 mice, dysregulation of IL-6 amplifier is observed (Non-Patent Literature 1). Dysregulation of IL-6 amplifier observed in F759 mice is believed to be caused as follows: SOCS3 protein signal transduction, which is dependent on the phosphorylation status of tyrosine residue 759 (Y759) of gp130 protein, is inhibited by F759 mutation, thereby decreasing the action of suppressing STAT3 signals via SOCS3 protein to enhance STAT3-dependent signals. It is known that autoimmune arthritis develops in almost 100% of the mice one to one-and-a-half years after birth. It is also known that arthritis develops earlier by injecting inflammatory cytokines, such as IL-6 and IL-17, into joints (e.g., malleolus joints) of the mice. Autoimmune arthritis in mice is believed to be a disease model for human rheumatoid arthritis.

### Example 4

The effect of administration of an ErbB1 protein inhibitor on lesion part in cytokine-induced arthritis mice was evaluated.

### Experimental Method

On each of days 0, 1, and 2, 100 ng of mIL-6 protein and 100 ng of mIL-17A protein, as cytokines, were injected into the malleolus joints of 8-week-old F759 mice to induce arthritis. At the same time, on each of days 0 to 11, 10 micrograms of PD168393 dissolved in DMSO was injected into the malleolus joints (n=4). Mice injected with the cytokines and DMSO only (n=4), and mice injected with DMSO only (n=4) served as control groups.

From day 0 to day 23, symptom of arthritis was evaluated by clinical scores by the same method as in Example 3. Fig. 15 (B) shows the evaluation results. It was observed that symptom of cytokine-induced arthritis was alleviated, or worsening of symptom of cytokine-induced arthritis was suppressed by administration of PD168393, an ErbB1 protein inhibitor.

### Example 5

The effects of administration of shRNA targeting epiregulin gene on lesion part in cytokine-induced arthritis mice were evaluated.

### Experimental Method

On each of days 0, 2, and 4, 3.8 × 10⁵ TU (Total transducing units needed) of lentivirus that expresses shRNA targeting epiregulin gene (Sigma-Aldrich Co. LLC., TRCN0000250419 (sh #1), TRCN0000250420 (sh #2), or TRCN0000250421 (sh #3)) dissolved in physiological saline was injected into the malleolus joints of 8-week-old F759 mice. Subsequently, on each of days 6, 7, and 8, 100 ng of mIL-6 protein and 100 ng of mIL-17A protein, as cytokines, dissolved in physiological saline were injected into the malleolus joints to induce arthritis (n=4). Mice injected with 3.8×10⁵ TU of nonspecific shRNA (Non-Target sh, Sigma-Aldrich Co. LLC.) instead of shRNA targeting epiregulin gene (n=3), mice injected with shRNA targeting NFκ-B p65 gene (p65 sh, Sigma-Aldrich Co. LLC., TRCN0000055346) instead of shRNA targeting epiregulin gene (n=3), and mice injected with physiological saline only (n=3) served as control groups.

TU (Total transducing units needed) was determined according to the information provided by Sigma-Aldrich Co. LLC. on each lentivirus vector lot (TU/ml).

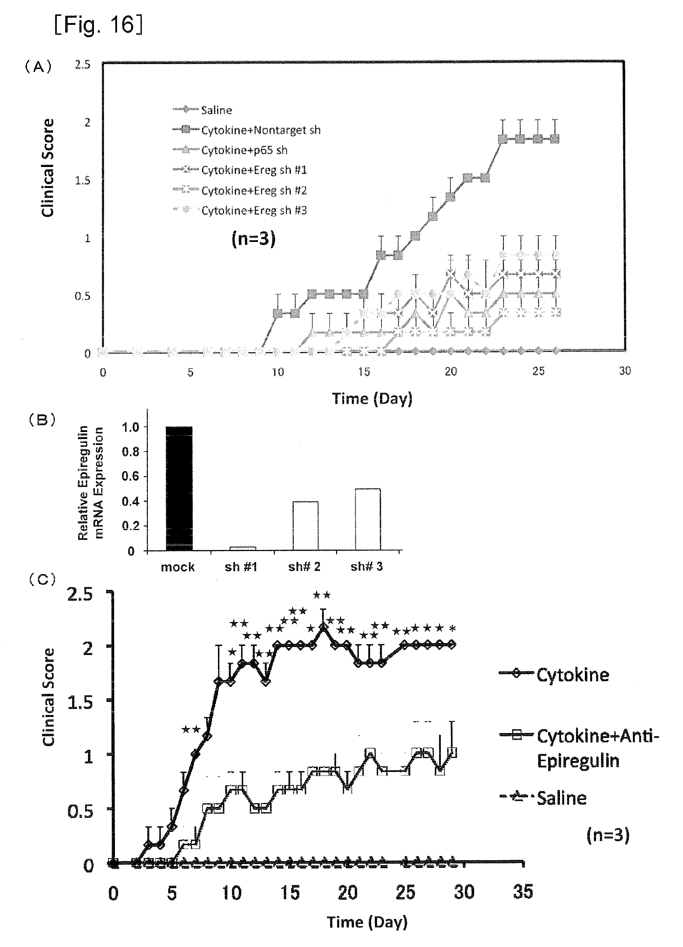

From day 0 to day 26, symptom of arthritis was evaluated by clinical scores by the same method as in Example 3. Fig. 16 (A) shows the evaluation results. It was observed that symptom of cytokine-induced arthritis was alleviated, or worsening of symptom of cytokine-induced arthritis was suppressed by administration of shRNA targeting epiregulin gene, to an extent similar to the case in which shRNA targeting NFκ-B p65 gene known to constitute IL-6 amplifier was administered.

Fig. 16 (B) shows the verification results of the gene expression suppression effects of the shRNA used. The expression amounts of epiregulin gene were compared between each sample obtained by introducing a 3-microliter amount of shRNA (sh #1: 4.8×10⁴ TU, sh #2: 5.4×10⁴ TU, sh #3: 6.0×10⁴ TU) into BC-1 cells by lentivirus carrying the shRNA and the control sample into which no shRNA was introduced, by a real-time RT-PCR method in the same manner as in Reference Example 2.

### Example 6

The effect of administration of anti-epiregulin antibody on lesion part in cytokine-induced arthritis mice was evaluated.

### Experimental Method

On each of days 0, 1, and 2, 100 ng of mIL-6 protein and 100 ng of mIL-17A protein, as cytokines, were injected into the malleolus joints of 8-week-old F759 mice to induce arthritis. At the same time, on each of days 0 to 14, 1 microgram of rat-derived anti-epiregulin antibody (R&D Systems, Inc., Cat# MAB1068) was injected into the malleolus joints (n=3). Mice injected with the cytokines only (n=3), and mice injected with physiological saline only (n=3) served as control groups.

From day 0 to day 29, symptom of arthritis was evaluated by clinical scores by the same method as in Example 3. Fig. 16 (C) shows the evaluation results. It was observed that symptom of cytokine-induced arthritis was alleviated, or worsening of symptom of cytokine-induced arthritis was suppressed by administration of the anti-epiregulin antibody.

The anti-epiregulin antibody (R&D Systems, Inc., Cat# MAB1068) used in this example has activity of neutralizing epiregulin protein-induced cell proliferation in mouse embryonic fibroblasts derived from Balb/3T3 mice. Specifically, the anti-epiregulin antibody is believed to inhibit the bond of ErbB1 protein with epiregulin protein.

### Example 7

The effect of administration of anti-epiregulin antibody in experimental autoimmune encephalomyelitis (EAE) mice was evaluated.

### Experimental Method

### (i) Preparation of EAE mice

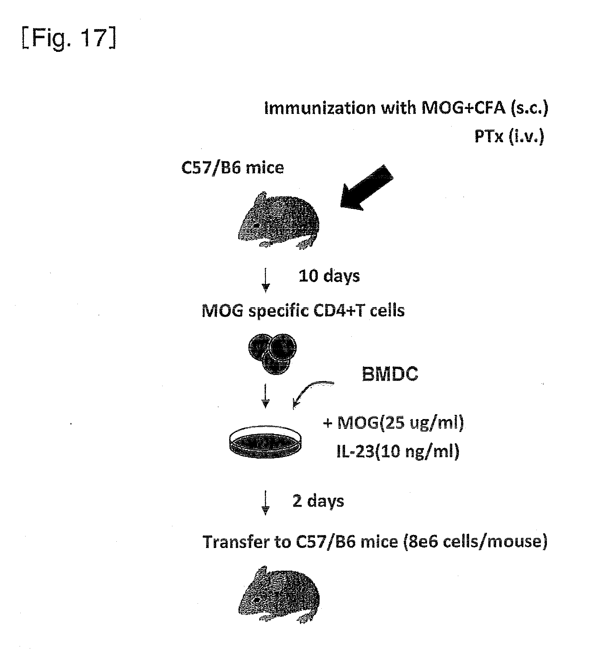

EAE mice were made according to the method described in Non-Patent Literature 1. Fig. 17 shows an outline.

200 micrograms of MOG (myelin oligodendrocyte glycoprotein) peptide (peptide comprising residues 33 to 55 of the full-length amino acid sequence of mouse MOG protein (amino acid sequence shown in SEQ ID NO: 9)) dissolved in Complete Freund's adjuvant (CFA) was administered to the tail of a 7- to 8-week-old C57/B6-strain mouse by subcutaneous injection, followed by intravenous injection of 200 nanograms of pertussis toxin (PTx).

A MOG-specific CD4⁺T cell group was obtained according to a ordinary method from the spleen removed after feeding the above mouse for 10 days. 2×10⁶ cells of the MOG-specific CD4⁺T cells were cultured with 5×10⁵ C57/B6-strain mouse bone marrow-derived dendritic cells (BMDCs) pulsed with MOG peptide in an RPMI 1640 medium containing 10% FBS in the presence of 20 ng/ml of recombinant IL-23 (rIL-23) protein in an incubator at a temperature of 37°C to obtain a cell group containing pathogenic Th17 cells.

The obtained cell group (8×10⁶ cells) was administered by intravenous injection to 7- to 8-week-old C57/B6-strain mice irradiated with a sublethal dose (5 Gy) of γ rays, followed by intravenous injection of 200 nanograms of pertussis toxin to obtain EAE mice (day 0).

### (ii) Administration of anti-epiregulin antibody

On days 0, 4, and 7, physiological saline containing 100 micrograms of rat-derived anti-epiregulin antibody (R&D Systems, Inc., Cat# MAB1068) was administered by intraperitoneal injection to the EAE mice obtained in the above (i) (n=4). EAE mice given a solution containing 100 micrograms of nonspecific IgG (Sigma-Aldrich Co. LLC.) in the same manner served as a control group (n=4).

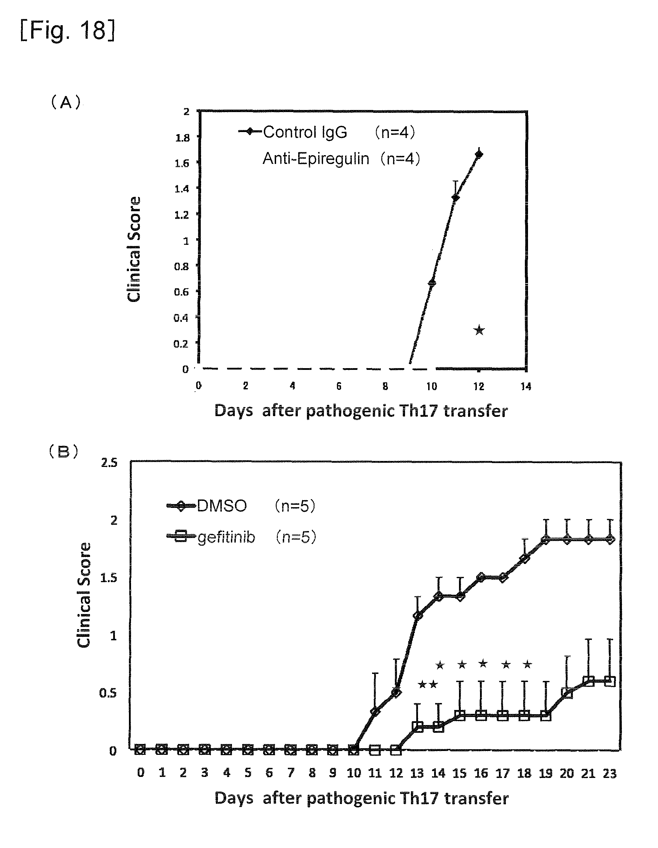

From day 0 to day 12, the clinical score in each mouse was evaluated according to the method described in Non-Patent Literature 1. Fig. 18 (A) shows the results. It was observed that symptom of experimental autoimmune encephalomyelitis was alleviated, or worsening of symptom of experimental autoimmune encephalomyelitis was suppressed by administration of the anti-epiregulin antibody.

Note that experimental autoimmune encephalomyelitis (EAE) mice are believed to be a disease model for human multiple sclerosis.

### Example 8

The effect of administration of gefitinib (product name: Iressa (registered trademark), AstraZeneca K.K.) in EAE mice (model mice for multiple sclerosis) was evaluated.

### Experimental Method

### (i) Preparation of EAE mice

EAE mice were obtained by the same method as in Example 7.

### (ii) Administration of gefitinib

On each of days 0 to 6, 5 mg of gefitinib dissolved in DMSO was administered by intraperitoneal injection to the EAE mice obtained in the above (i) (n=5). EAE mice given only solvent DMSO in the same manner served as a control group (n=5).

From day 0 to day 13, the clinical score in each mouse was evaluated according to the method described in Non-Patent Literature 1. Fig. 18 (B) shows the results. It was observed that symptom of experimental autoimmune encephalomyelitis was alleviated, or worsening of symptom of experimental autoimmune encephalomyelitis was suppressed by administration of gefitinib.

### Example 9

The effects of administration of shRNA targeting ErbB1 gene on lesion part in cytokine-induced arthritis mice were evaluated.

### Experimental Method

The effects of administration of shRNA targeting ErbB1 on lesion part were evaluated in cytokine-induced arthritis mice in the same manner as in Example 5, except that lentivirus that expresses shRNA targeting ErbB1 (Egfr) gene (Sigma-Aldrich Co. LLC., TRCN0000023480 (sh #1), TRCN0000023481 (sh #2), or TRCN0000023483 (sh #3)) was administered instead of the lentiviruses that express shRNA targeting epiregulin gene.

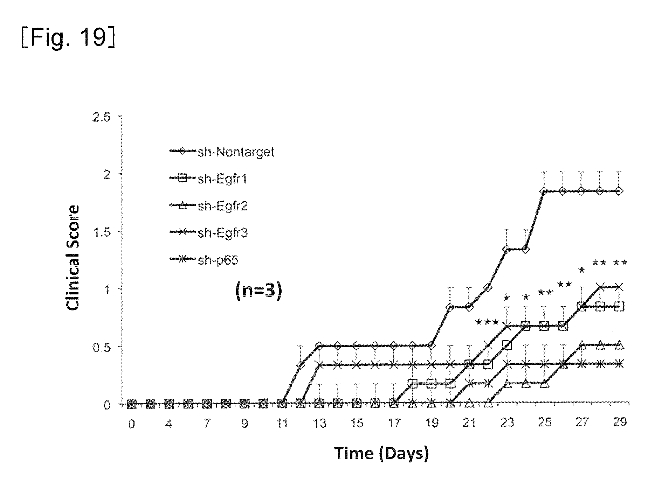

From day 0 to day 29, symptom of arthritis was evaluated by clinical scores by the same method as in Example 3. Fig. 19 shows the evaluation results. It was observed that symptom of cytokine-induced arthritis was alleviated, or worsening of symptom of cytokine-induced arthritis was suppressed by administration of shRNA targeting ErbB1 (Egfr) gene, to an extent similar to the case in which shRNA targeting NFκ-B p65 gene known to constitute IL-6 amplifier was administered.

### Example 10

The effect of administration of gefitinib (product name: Iressa (registered trademark), AstraZeneca K.K.) on lesion part in cytokine-induced arthritis mice was evaluated.

### Experimental Method

On each of days 0, 1, and 2, 100 ng of mIL-6 protein and 100 ng of mIL-17A protein, as cytokines, were injected into the malleolus joints of 8-week-old F759 mice to induce arthritis. At the same time, on each of days 0 to 23, 10 micrograms of gefitinib dissolved in DMSO was injected into the malleolus joints (n=4). Mice injected with the cytokines and DMSO only (n=4), and mice injected with DMSO only (n=4) served as control groups.

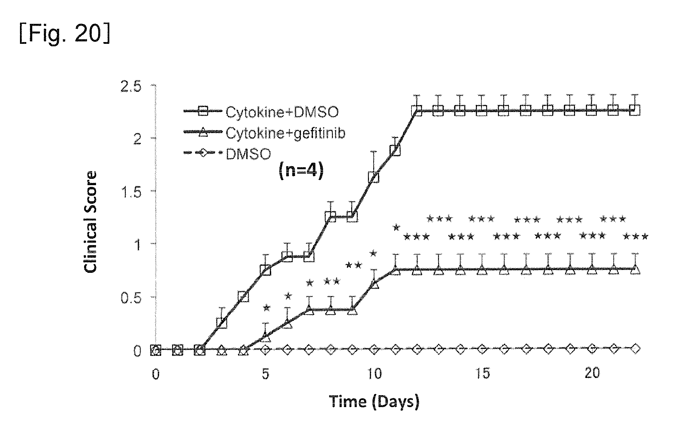

From day 0 to day 23, symptom of arthritis was evaluated by clinical scores by the same method as in Example 3. Fig. 20 shows the evaluation results. It was observed that symptom of cytokine-induced arthritis was alleviated, or worsening of symptom of cytokine-induced arthritis was suppressed by administration of gefitinib.

### Example 11

Decrease in the expression amount of IL-6 protein due to suppression of epiregulin expression in the activation of IL-6 amplifier was detected.

### Experimental Method

Lentivirus that expresses shRNA targeting epiregulin gene (Sigma-Aldrich Co. LLC., TRCN0000250421) was introduced into mouse BC-1 cells according to a ordinary method, and cells into which the lentivirus carrying shRNA was introduced were selected by puromycin selection.

Subsequently, 2×10⁵ cells of the obtained clone and 2×10⁵ cells of the control clone (lentivirus that expresses nonspecific shRNA) were separately cultured for 48 hours or 72 hours under each of the conditions (e) to (h) of Reference Example 2, or under each of the conditions (e) to (h) of Reference Example 2 with the addition of 100 ng/ml of mouse epiregulin protein in each of the conditions.

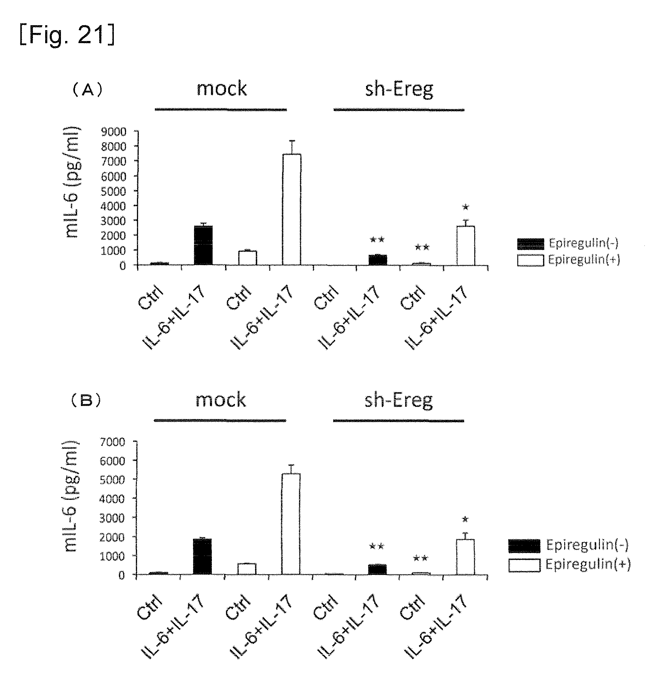

After the culture, the production amount of mIL-6 protein under each condition was quantified by an ELISA method in the same manner as in Reference Example 1. Fig. 21 shows the results. Decrease in the production amount of mIL-6 protein by the effect of shRNA targeting epiregulin gene was measured in both the condition in which mouse epiregulin protein was added, and the condition in which mouse epiregulin protein was not added.

### Discussion

Reference Examples 1 and 2 suggest that ErbB1 protein and growth factors contribute to the activation of IL-6 amplifier. Regarding the growth factors, Reference Examples 3 and 4 suggest that growth factors (ligands) in ErbB1 pathway specifically contribute to the activation of IL-6 amplifier. Further, Reference Example 6 suggests that epiregulin contributes to positive feedback control in IL-6 amplifier. In addition, Reference Example 5 and Example 1 suggest that ErbB1 receptor is specifically involved in the activation of IL-6 amplifier. Reference Examples 7 and 9 to 11 indicate that a similar mechanism exists in humans.

As shown in the results of Examples 1 and 2, it was indicated that the IL-6 production induced by the activation of IL-6 amplifier by the addition of hIL-6 protein, hIL-6R protein, and mIL-17A protein can be suppressed by ErbB1 inhibitor PD153065 or PD168393, or PI3 kinase inhibitor LY294002. These results also suggest that a compound that inhibits the function of a protein belonging to ErbB1 pathway functions as an IL-6 amplifier inhibitor, i.e., an agent for suppressing the production of inflammatory cytokines. Further, it is suggested that such a compound functions as an immunosuppressant.

The results of Examples 3 to 10 show that a compound that inhibits expression of the function of a protein in ErbB1 pathway can alleviate symptom of rheumatoid arthritis or multiple sclerosis in model mice of such diseases. Additionally, Reference Example 7 suggests that IL-6 amplifier is activated to promote inflammatory cytokine production in affected part of rheumatoid arthritis. Further, the results of Reference Example 8 suggest that dysregulation of IL-6 amplifier contributes to the development of atopic dermatitis. Thus, a compound that inhibits expression of the function of a protein in ErbB1 pathway can be used as a therapeutic agent or a prophylactic agent for diseases such as autoimmune diseases, inflammatory diseases, and allergic diseases, and symptoms accompanying organ transplants.

Of proteins in ErbB1 pathway, epiregulin protein contributes to the activation of IL-6 amplifier, and the activation of IL-6 amplifier increases the expression amount of epiregulin gene. This fact suggests that epiregulin contributes to positive feedback control in IL-6 amplifier. Specifically, a compound that inhibits expression of the function of epiregulin protein is presumed to be particularly highly effective as a compound that suppresses the activation of IL-6 amplifier and an active ingredient of a therapeutic agent or prophylactic agent for diseases such as autoimmune diseases, inflammatory diseases, and allergic diseases, and symptoms accompanying organ transplants.

Further, the results of Examples 1 to 10 suggest that a therapeutic agent or prophylactic agent for diseases such as inflammatory diseases and allergic diseases, and symptoms accompanying organ transplants is screened by screening a compound that inhibits expression of the function of a protein in ErbB1 pathway. Example 11 demonstrates means usable in a screening method suitable for determining whether a compound suppresses inflammatory cytokine expression.

## Claims

1. A therapeutic agent or a prophylactic agent for at least one disease or symptom selected from the group consisting of autoimmune diseases, inflammatory diseases, allergic diseases, and symptoms accompanying organ transplants, the therapeutic agent or the prophylactic agent comprising a compound that inhibits expression of the function of a protein belonging to ErbB1 pathway.

2. The therapeutic agent or the prophylactic agent according to claim 1, wherein the protein belonging to ErbB1 pathway is at least one member selected from the group consisting of ErbB1 protein and a ligand protein of ErbB1 protein.

3. The therapeutic agent or the prophylactic agent according to claim 1 or 2, wherein the protein belonging to ErbB1 pathway is epiregulin.

4. The therapeutic agent or the prophylactic agent according to any one of claims 1 to 3, wherein the compound that inhibits expression of the function of the protein belonging to ErbB1 pathway is an antibody that specifically binds to the protein.

5. The therapeutic agent or the prophylactic agent according to any one of claims 1 to 3, wherein the compound that inhibits the function of the protein belonging to ErbB1 pathway is at least one RNA molecule selected from the group consisting of siRNA, shRNA, and miRNA that targets a gene encoding the protein or a vector capable of expressing the RNA molecule.

6. The therapeutic agent or the prophylactic agent according to claim 1 or 2, wherein the compound that inhibits the function of the protein belonging to ErbB1 pathway is a kinase inhibitor specific to ErbB1 protein.

7. The therapeutic agent or the prophylactic agent according to claim 6, wherein the compound that inhibits the function of the protein belonging to ErbB1 pathway is at least one member selected from the group consisting of PD153035, PD168393, gefitinib, and erlotinib.

8. The therapeutic agent or the prophylactic agent according to any one of claims 1 to 7, wherein the disease or symptom is an autoimmune disease.

9. The therapeutic agent or the prophylactic agent according to any one of claims 1 to 8, wherein the autoimmune disease is at least one member selected from the group consisting of rheumatoid arthritis and multiple sclerosis.

10. A method for screening, among test substances, an active ingredient of a therapeutic agent or a prophylactic agent for at least one disease or symptom selected from the group consisting of autoimmune diseases, inflammatory diseases, allergic diseases, and symptoms accompanying organ transplants, the method comprising the steps of:
(i) determining whether a test substance is a compound that inhibits expression of the function of a protein belonging to ErbB1 pathway; and
(ii) selecting the test substance determined as the compound that inhibits expression of the function of the protein belonging to ErbB1 pathway in Step (i) as the active ingredient of the therapeutic agent or the prophylactic agent.

11. The method according to claim 10, wherein the protein belonging to ErbB1 pathway is at least one member selected from the group consisting of ErbB1 protein and a ligand protein of ErbB1 protein.

12. The method according to claim 10 or 11, wherein the protein belonging to ErbB1 pathway is epiregulin.

13. The method according to any one of claims 10 to 12, wherein the disease or symptom is an autoimmune disease.

14. The method according to any one of claims 10 to 13, wherein the autoimmune disease is at least one member selected from the group consisting of rheumatoid arthritis and multiple sclerosis.

15. The method according to any one of claims 10 to 14, wherein the test substances are either an antibody or a nucleic acid capable of suppressing expression of the protein.
